# EUROPEAN PATENT SPECIFICATION

(11) **EP 3 285 814 B1**
(45) Date of publication and mention of the grant of the patent: **24.06.2020**
(21) Application number: 16719682.3
(22) Date of filing: 20.04.2016
(51) Int. Cl.: A61K 51/04, A61K 51/12, A61P 35/00

(54) **IMAGING OF TUMOR-ASSOCIATED MACROPHAGES**
BILDGEBUNG VON TUMORASSOZIIERTEN MAKROPHAGEN
IMAGERIE DE MACROPHAGES ASSOCIÉS À UNE TUMEUR

(30) Priority: 20.04.2015 US 201562150104 P
(43) Date of publication of application: 28.02.2018
(73) Proprietor: Memorial Sloan-Kettering Cancer Center, New York, NY 10065 (US)
(72) Inventor: PEREZ-MEDINA, Carlos, New York, New York 10065 (US); REINER, Thomas, New York, New York 10065 (US); LEWIS, Jason S., New York, New York 10065 (US); MULDER, Willem, J.M., New York, New York 10029 (US); FAYAD, Zahi A., New York, New York 10029 (US); FISHER, Edward, New York, New York 10016 (US)
(74) Representative: Regimbeau
(86) International application number: PCT/US2016/028349
(87) International publication number: WO 2016/172146

(56) References cited:
- WO-A2-2011/044545
- US-A1- 2009 110 739
- US-A1- 2013 330 274
- SCHOUTEN D ET AL: "Development of lipoprotein-like lipid particles for drug targeting: neo-high density lipoproteins", MOLECULAR PHARMACOLOGY, AMERICAN SOCIETY FOR PHARMACOLOGY AND EXPERIMENTAL THERAPEUTICS, US, vol. 44, 1 January 1993 (1993-01-01), pages 486-492, XP008118882, ISSN: 0026-895X
- JUAN C. FRIAS ET AL: "Recombinant HDL-Like Nanoparticles: A Specific Contrast Agent for MRI of Atherosclerotic Plaques", JOURNAL OF THE AMERICAN CHEMICAL SOCIETY, vol. 126, no. 50, 1 December 2004 (2004-12-01), pages 16316-16317, XP55163160, ISSN: 0002-7863, DOI: 10.1021/ja044911a
- CORSICO B ET AL: "Evidence for a central apolipoprotein A-I domain loosely bound to lipids in discoidal lipoproteins that us capable of penetrating the bilayer of phospholipid vesicles", JOURNAL OF BIOLOGICAL CHEMISTRY, AMERICAN SOCIETY FOR BIOCHEMISTRY AND MOLECULAR BIOLOGY, US, vol. 276, no. 20, 18 May 2001 (2001-05-18) , pages 16978-16985, XP002250374, ISSN: 0021-9258, DOI: 10.1074/JBC.M011533200
- CORBIN I R ET AL: "Enhanced Cancer-Targeted Delivery Using Engineered High-Density Lipoprotein-Based Nanocarriers", JOURNAL OF BIOMEDICAL NANOTECHNOLOGY, AMERICAN SCIENTIFIC PUBLISHERS, US, vol. 3, no. 4, 1 December 2007 (2007-12-01), pages 367-376, XP008137630, ISSN: 1550-7033
- MOOBERRY L ET AL: "Receptor mediated uptake of paclitaxel from a synthetic high density lipoprotein nanocarrier", JOURNAL OF DRUG TARGETING, HARWOOD ACADEMIC PUBLISHERS GMBH, DE, vol. 18, no. 1, 1 January 2010 (2010-01-01), pages 53-58, XP008165075, ISSN: 1061-186X, DOI: 10.3109/10611860903156419

## Description

### Cross Reference to Related Application

This application claims the benefit of U.S. Application Serial No. 62/150,104 filed on April 20, 2015.

### Technical Field

This invention relates generally to compositions and methods for imaging tumors. More particularly, in certain embodiments, the invention relates to using lipoprotein nanoparticles for imaging tumor-assisted macrophages (TAMs).

### Government Funding

This invention was made with government support under Grant Numbers. K25 EB016673 (T.R.), R01 HL118440 (W.J.M.M.), R01 HL125703 (W.J.M.M.) and R01 CA155432 (W.J.M.M.), awarded by the National Institutes of Health (NIH). The government has certain rights in this invention.

### Background

Tumor-associated macrophage (TAM) immunology has become an active research field in recent years. TAMs are considered a promising target for better and tailored treatment of malignant growths. The role of TAMs in different stages of tumor development and in therapy is starting to be established. In ovarian cancer, for instance, it has been shown that TAMs contribute to tumor progression, whereas in breast cancer (among others), TAM infiltration has been linked with poor prognosis.

TAMs are a type of blood-borne phagocytes, derived from circulating monocytes or resident tissue macrophages. Their complex role in carcinogenesis generally leads to disease progression in many cancers, which share some similar pathological mechanisms. Thus, high TAM burden has often been associated with poor prognosis. During cancer progression, circulating monocytes and macrophages are recruited to tumors where they differentiate under the influence of a milieu of growth factors and cytokines. In this process, TAMs themselves become critical modulators of the tumor microenvironment because they foster tumor growth, immune suppression, metastasis and chemoresistance by generating tumor-promoting conditions. The implication of TAMs in modulating the immune system response to tumor growth has led to TAM-targeting therapies.

Imaging of macrophages to monitor inflammatory response has been an active area of research. To this end, several nanoparticulate materials have been developed as TAM imaging agents. Among others, several iron oxide-based MRI probes have been applied, as well as ⁶⁴Cu-labeled/mannose-functionalized liposomes, and nanobodies. WO2011044545 discloses nanoparticles comprising radiolabelled phospholipids and apolipoprotein A1 for imaging and diagnostic methods. B. Corsico et al. (Journal of Biological Chemistry, 276(2), pages 16978-16985) and D. Schouten et al. (Molecular Pharmacology, 44: pages 486-492) disclose ¹²⁵I-labelled apolipoprotein in nanoparticles for targeted delivery. Although TAMs also have high diagnostic and prognostic value, TAM imaging still remains largely unexplored.

Thus, there is a need for improved systems and methods of assessing prognosis and therapy outcome of different stages of tumor development. There is also a need for specific and quantifiable TAM imaging agents, particularly for non-invasive monitoring of TAM immunology and targeted treatment.

### Summary

The invention is defined by the claims. Any subject-matter falling outside the scope of the claims is provided for information purposes only.

Presented herein are methods and compositions for non-invasive imaging of TAMs with discoidal high-density lipoproteins to assess prognosis and therapy outcome. TAMs are increasingly investigated in cancer immunology, and are considered a promising target for better and tailored treatment of malignant growths. Although TAMs also have high diagnostic and prognostic value, TAM imaging still remains largely unexplored. Imaging agents/methods provided herein are of value for non-invasive *in vivo* evaluation of TAM burden, not only in preclinical but also in clinical settings.

Thus, in certain embodiments, the invention provides compositions and methods that use high density lipoprotein (HDL) nanoparticles for imaging of TAMs. The HDL molecules can be labeled with radioisotopes and/or fluorophores for *in vivo* visualization by PET and/or near-infrared fluorescence imaging.

In one aspect, the invention is directed to a composition comprising a discoidal high density lipoprotein nanoparticle, the nanoparticle comprising Apolipoprotein 1 (ApoA1) and one or more phospholipids, wherein the nanoparticle is radiolabeled with a radioisotope via incorporation of a chelator-modified ApoA1 with a radioisotope; and optionally incorporation of a chelator-modified phospholipid with a radioisotope (e.g., wherein from 0.5 wt.% to 1.5 wt.% of the nanoparticle is chelator-modified phospholipid).

In certain embodiments, the nanoparticle is radiolabeled with the radioisotope via the chelator-modified ApoA1, and the nanoparticle comprises the chelator-modified phospholipid.

In certain embodiments, the nanoparticle has a molecular weight within a range from 100 kDa to 400 kDa as measured by size exclusion chromatography.

In certain embodiments, the discoidal high density lipoprotein nanoparticle has average diameter within a range from 5 nm to 30 nm as measured by DLS in water or phosphate-buffered saline (PBS).

In certain embodiments, the one or more phospholipids comprise one or more members selected from the group consisting of dimyristoyl phosphatidyl choline (DMPC), 1,2-dipentadecanoyl-sn-glycero-3 -phosphocholine, 1,2-dipalmitoyl-sn-glycero-3 -phosphocholine (DPPC), 1,2-diheptadecanoyl-sn-glycero-3 -phosphocholine, 1,2-distearoyl-sn-glycero-3-phosphocholine (DSPC), 1-myristoyl-2-hydroxy-sn-glycero-3-phosphocholine (MHPC), 1-pentadecanoyl-2-hydroxy-sn-glycero-3-phosphocholine, 1-palmitoyl-2-hydroxy-sn-glycero-3-phosphocholine (PHPC), 1-heptadecanoyl-2-hydroxy-sn-glycero-3-phosphocholine, 1-stearoyl-2-hydroxy-sn-glycero-3-phosphocholine (SHPC), 1-oleoyl-2-hydroxy-sn-glycero-3-phosphocholine (OHPC), 1,2-dioleoyl-sn-glycero-3-phosphocholine (DOPC), and 1,2-dipalmitoleoyl-sn-glycero-3-phosphocholine. In certain embodiments, the chelator-modified phospholipid comprises 1,2-distearoyl-sn-glycero-3-phosphoethanolamine (DSPE).

In certain embodiments, some of the one or more phospholipids in the nanoparticle are effectively replaced by the chelator-modified phospholipid.

In certain embodiments, composition comprises a carrier.

Also disclosed is a method of imaging tumor-associated macrophages (TAMs) by high density lipoprotein positron emission tomography (HDL PET), the method comprising: administering to a subject a composition comprising the discoidal high density lipoprotein nanoparticle of any one of the preceding claims to allow the nanoparticle to accumulate in a region of TAMs; and detecting the radioisotope via PET after accumulation of the nanoparticle in the region of TAMs.

In another aspect, the invention is directed to a method of making the discoidal high density lipoprotein nanoparticle of claim 1, the method comprising one or more of (i), (ii), and optionally (iii) and/or (iv): (i) modifying ApoAl with a chelator, reacting the modified ApoAl with a radioisotope comprising an oxalate moiety (or other moiety such that the radioisotope comprises Zr in its Zr(IV) state), and incorporating the radiolabeled chelator-modified ApoA1 in the nanoparticle; (ii) modifying ApoA1 with a chelator, incorporating the chelator-modified ApoA1 in the nanoparticle, and reacting the chelator-modified ApoAl with a radioisotope comprising an oxalate moiety (or other moiety such that the radioisotope comprises Zr in its Zr(IV) state); (iii) modifying DSPE (or other phospholipid) with a chelator, reacting the modified DSPE (or other phospholipid) with a radioisotope comprising an oxalate moiety (or other moiety such that the radioisotope comprises Zr in its Zr(IV) state), and incorporating the radiolabeled chelator-modified DSPE (or other phospholipid) in the nanoparticle; and (iv) modifying DSPE (or other phospholipid) with a chelator, incorporating the chelator-modified DSPE (or other phospholipid) in the nanoparticle, and reacting the chelator-modified DSPE (or other phospholipid) with a radioisotope comprising an oxalate moiety (or other moiety such that the radioisotope comprises Zr in its Zr(IV) state).

In certain embodiments, ApoAl is modified with the chelator, wherein the modifed ApoA1 is reacted with the radioisotope comprising an oxalate moiety (or other moiety such that the radioisotope comprises Zr in its Zr(IV) state), and wherein the radiolabeled chelator-modified ApoA1 is incorporated in the nanoparticle.

In certain embodiments, ApoA1 in the nanoparticle is modified with a chelator, wherein the chelator-modified ApoA1 is incorporated in the nanoparticle, and wherein the chelator-modified ApoA1 is reacted with the radioisotope comprising the oxalate moiety (or other moiety such that the radioisotope comprises Zr in its Zr(IV) state).

In certain embodiments, DSPE (or other phospholipid) is modified with the chelator, wherein the modified DSPE (or other phospholipid) is reacted with the radioisotope comprising the oxalate moiety (or other moiety such that the radioisotope comprises Zr in its Zr(IV) state), and wherein the radiolabeled chelator-modified DSPE (or other phospholipid) is incorporated in the nanoparticle.

In certain embodiments, DSPE (or other phospholipid) is modified with the chelator, wherein the chelator-modified DSPE (or other phospholipid) is incorporated in the nanoparticle, and wherein the chelator-modified DSPE (or other phospholipid) is reacted with the radioisotope comprising the oxalate moiety (or other moiety such that the radioisotope comprises Zr in its Zr(IV) state).

In certain embodiments, the radioisotope comprises a member selected from the group consisting of ⁸⁹Zr, ^{99m}Tc, ¹¹¹In, ⁶⁴Cu, ⁶⁷Ga, ¹⁸⁶Re, ¹⁸⁸Re, ¹⁵³Sm, ¹⁷⁷Lu, ⁶⁷Cu, ¹²³I, ¹²⁴I, ¹²⁵I, ¹¹C, ¹³N, ¹⁵O, ¹⁸F, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, and ¹⁹²Ir. In certain embodiments, the radioisotope comprises ⁸⁹Zr.

In certain embodiments, the chelator is a member selected from the group consisting of deferoxamine B (DFO), 1,4,8,1 1-tetraazabicyclo[6.6.2]hexadecane-4,11-diyl)diacetic acid (CB-TE2A); diethylenetriaminepentaacetic acid (DTPA); 1,4,7, 10-tetraazacyclotetradecane-1,4,7, 10-tetraacetic acid (DOTA); thylenediaminetetraacetic acid (EDTA); ethylene glycolbis(2-aminoethyl)-N,N,N',N'- tetraacetic acid (EGTA); 1,4,8,1 1-tetraazacyclotetradecane-1,4,8,11-tetraacetic acid (TETA); ethylenebis-(2-4 hydroxy-phenylglycine) (EHPG); 5-Cl-EHPG; 5Br-EHPG; 5- Me-EHPG; 5t-Bu-EHPG; 5-sec-Bu-EHPG; benzodiethylenetriamine pentaacetic acid (benzo-DTPA); dibenzo-DTPA; phenyl-DTPA, diphenyl-DTPA; benzyl-DTPA; dibenzyl DTPA; bis-2 (hydroxybenzyl)-ethylene-diaminediacetic acid (HBED) and derivatives thereof; Ac-DOTA; benzo-DOTA; dibenzo-DOTA; 1,4,7-triazacyclononane N,N',N"- triacetic acid (NOTA); benzo-NOTA; benzo-TETA, Octadentate Hydroxypyridinonate (HOPO) ligands (e.g., 3,4,3-(LI-1,2-HOPO)), benzo-DOTMA, where DOTMA is 1,4,7, 10-tetraazacyclotetradecane-1,4,7,10-tetra(methyl tetraacetic acid), benzo-TETMA (e.g., wherein TETMA is 1,4,8,11-tetraazacyclotetradecane-1,4,8,1 1-(methyl tetraacetic acid)); derivatives of 1,3-propylenediaminetetraacetic acid (PDTA); triethylenetetraaminehexaacetic acid (TTHA); derivatives of 1,5,10-N,N',N"-tris(2,3- dihydroxybenzoyl)-tricatecholate (LICAM); and 1,3,5-N,N',N"-tris(2,3- dihydroxybenzoyl)aminomethylbenzene (MECAM), and other metal chelators. In certain embodiments, the chelator comprises deferoxamine B (DFO).

In certain embodiments, from 0.5 wt.% to 1.5 wt.% of the nanoparticle is a radiolabeled chelator-modified phospholipid.

In certain embodiments, not all of the DSPE-DFO incorporated into the nanoparticle is radiolabeled.

In certain embodiments, DSPE-DFO is in substantial excess.

In another aspect, the invention is directed to a composition comprising a discoidal high density lipoprotein nanoparticle (e.g., having molecular weight within a range from 100 kDa to 400 kDa measured by size exclusion chromatography, and having average diameter within a range from 5 nm to 30 nm as measured by DLS in water or phosphate-buffered saline (PBS)), the nanoparticle comprising Apolipoprotein 1 (ApoA1) and one or more phospholipids (e.g., dimyristoyl phosphatidyl choline (DMPC), 1,2-dipentadecanoyl-sn-glycero-3-phosphocholine, 1,2-dipalmitoyl-sn-glycero-3-phosphocholine (DPPC), 1,2-diheptadecanoyl-sn-glycero-3-phosphocholine, 1,2-distearoyl-sn-glycero-3-phosphocholine (DSPC), 1-myristoyl-2-hydroxy-sn-glycero-3-phosphocholine (MHPC), 1-pentadecanoyl-2-hydroxy-sn-glycero-3-phosphocholine, 1-palmitoyl-2-hydroxy-sn-glycero-3-phosphocholine (PHPC), 1-heptadecanoyl-2-hydroxy-sn-glycero-3-phosphocholine, 1-stearoyl-2-hydroxy-sn-glycero-3-phosphocholine (SHPC), 1-oleoyl-2-hydroxy-sn-glycero-3-phosphocholine (OHPC), 1,2-dioleoyl-sn-glycero-3-phosphocholine (DOPC), and/or 1,2-dipalmitoleoyl-sn-glycero-3-phosphocholine), wherein the nanoparticle is radiolabeled with a radioisotope via incorporation of chelator-modified ApoA1 with a radioisotope; and optionally incorporation of chelator-modified phospholipid (e.g., 1,2-distearoyl-sn-glycero-3-phosphoethanolamine (DSPE)) with a radioisotope, wherein from 0.5 wt.% to 1.5 wt.% of the nanoparticle is chelator-modified phospholipid (e.g., DSPE) (e.g., some of the DMPC in the nanoparticle is effectively replaced by chelator-modified lipid DSPE) for use in a method of *in vivo* diagnosis of a disease (e.g., cancer) in a subject, wherein the *in vivo* diagnosis comprises: delivering the composition to the subject to allow the nanoparticle to accumulate in a region of TAMs; and detecting the radioisotope via PET after accumulation of the nanoparticle in the region of TAMs.

In another aspect, the invention is directed to a composition comprising a discoidal high density lipoprotein nanoparticle (e.g., having molecular weight within a range from 100 kDa to 400 kDa measured by size exclusion chromatography, and having average diameter within a range from 5 nm to 30 nm as measured by DLS in water or phosphate-buffered saline (PBS)), the nanoparticle comprising Apolipoprotein 1 (ApoA1) and one or more phospholipids (e.g., dimyristoyl phosphatidyl choline (DMPC), 1,2-dipentadecanoyl-sn-glycero-3-phosphocholine, 1,2-dipalmitoyl-sn-glycero-3-phosphocholine (DPPC), 1,2-diheptadecanoyl-sn-glycero-3-phosphocholine, 1,2-distearoyl-sn-glycero-3-phosphocholine (DSPC), 1-myristoyl-2-hydroxy-sn-glycero-3-phosphocholine (MHPC), 1-pentadecanoyl-2-hydroxy-sn-glycero-3-phosphocholine, 1-palmitoyl-2-hydroxy-sn-glycero-3-phosphocholine (PHPC), 1-heptadecanoyl-2-hydroxy-sn-glycero-3-phosphocholine, 1-stearoyl-2-hydroxy-sn-glycero-3-phosphocholine (SHPC), 1-oleoyl-2-hydroxy-sn-glycero-3-phosphocholine (OHPC), 1,2-dioleoyl-sn-glycero-3-phosphocholine (DOPC), and/or 1,2-dipalmitoleoyl-sn-glycero-3-phosphocholine), wherein the nanoparticle is radiolabeled with a radioisotope via incorporation of chelator-modified ApoA1 with a radioisotope; and optionally incorporation of chelator-modified phospholipid (e.g., 1,2-distearoyl-sn-glycero-3-phosphoethanolamine (DSPE)) with a radioisotope, wherein from 0.5 wt.% to 1.5 wt.% of the nanoparticle is chelator-modified phospholipid (e.g., DSPE) (e.g., some of the DMPC in the nanoparticle is effectively replaced by chelator-modified lipid DSPE) for use in a method of assessing prognosis and therapy outcome of different stages of a disease (e.g., tumor development) in a subject, wherein the assessing prognosis and therapy outcome comprises: delivering the composition to the subject to allow the nanoparticle to accumulate in a region of TAMs; and detecting the radioisotope via PET after accumulation of the nanoparticle in the region of TAMs.

In another aspect, the invention is directed to a composition comprising a discoidal high density lipoprotein nanoparticle (e.g., having molecular weight within a range from 100 kDa to 400 kDa measured by size exclusion chromatography, and having average diameter within a range from 5 nm to 30 nm as measured by DLS in water or phosphate-buffered saline (PBS)), the nanoparticle comprising Apolipoprotein 1 (ApoA1) and one or more phospholipids (e.g., dimyristoyl phosphatidyl choline (DMPC), 1,2-dipentadecanoyl-sn-glycero-3-phosphocholine, 1,2-dipalmitoyl-sn-glycero-3-phosphocholine (DPPC), 1,2-diheptadecanoyl-sn-glycero-3-phosphocholine, 1,2-distearoyl-sn-glycero-3-phosphocholine (DSPC), 1-myristoyl-2-hydroxy-sn-glycero-3-phosphocholine (MHPC), 1-pentadecanoyl-2-hydroxy-sn-glycero-3-phosphocholine, 1-palmitoyl-2-hydroxy-sn-glycero-3-phosphocholine (PHPC), 1-heptadecanoyl-2-hydroxy-sn-glycero-3-phosphocholine, 1-stearoyl-2-hydroxy-sn-glycero-3-phosphocholine (SHPC), 1-oleoyl-2-hydroxy-sn-glycero-3-phosphocholine (OHPC), 1,2-dioleoyl-sn-glycero-3-phosphocholine (DOPC), and/or 1,2-dipalmitoleoyl-sn-glycero-3-phosphocholine), wherein the nanoparticle is radiolabeled with a radioisotope via incorporation of chelator-modified ApoA1 with a radioisotope; and optionally incorporation of chelator-modified phospholipid (e.g., 1,2-distearoyl-sn-glycero-3-phosphoethanolamine (DSPE)) with a radioisotope, wherein from 0.5 wt.% to 1.5 wt.% of the nanoparticle is chelator-modified phospholipid (e.g., DSPE) (e.g., some of the DMPC in the nanoparticle is effectively replaced by chelator-modified lipid DSPE) for use in *in vivo* diagnosis.

In another aspect, the invention is directed to a composition comprising a discoidal high density lipoprotein nanoparticle (e.g., having molecular weight within a range from 100 kDa to 400 kDa measured by size exclusion chromatography, and having average diameter within a range from 5 nm to 30 nm as measured by DLS in water or phosphate-buffered saline (PBS)), the nanoparticle comprising Apolipoprotein 1 (ApoA1) and one or more phospholipids (e.g., dimyristoyl phosphatidyl choline (DMPC), 1,2-dipentadecanoyl-sn-glycero-3-phosphocholine, 1,2-dipalmitoyl-sn-glycero-3-phosphocholine (DPPC), 1,2-diheptadecanoyl-sn-glycero-3-phosphocholine, 1,2-distearoyl-sn-glycero-3-phosphocholine (DSPC), 1-myristoyl-2-hydroxy-sn-glycero-3-phosphocholine (MHPC), 1-pentadecanoyl-2-hydroxy-sn-glycero-3-phosphocholine, 1-palmitoyl-2-hydroxy-sn-glycero-3-phosphocholine (PHPC), 1-heptadecanoyl-2-hydroxy-sn-glycero-3-phosphocholine, 1-stearoyl-2-hydroxy-sn-glycero-3-phosphocholine (SHPC), 1-oleoyl-2-hydroxy-sn-glycero-3-phosphocholine (OHPC), 1,2-dioleoyl-sn-glycero-3-phosphocholine (DOPC), and/or 1,2-dipalmitoleoyl-sn-glycero-3-phosphocholine), wherein the nanoparticle is radiolabeled with a radioisotope via incorporation of chelator-modified ApoA1 with a radioisotope; and optionally incorporation of chelator-modified phospholipid (e.g., 1,2-distearoyl-sn-glycero-3-phosphoethanolamine (DSPE)) with a radioisotope, wherein from 0.5 wt.% to 1.5 wt.% of the nanoparticle is chelator-modified phospholipid (e.g., DSPE) (e.g., some of the DMPC in the nanoparticle is effectively replaced by chelator-modified lipid DSPE) for use in assessing prognosis and therapy outcome of different stages of a disease (e.g., tumor development).

In certain embodiments, the radioisotope comprises a member selected from the group consisting of ⁸⁹Zr, ^{99m}Tc, ¹¹¹In, ⁶⁴Cu, ⁶⁷Ga, ¹⁸⁶Re, ¹⁸⁸Re, ¹⁵³Sm, ¹⁷⁷Lu, ⁶⁷Cu, ¹²³I, ¹²⁴I, ¹²⁵I, ¹¹C, ¹³N, ¹⁵O, ¹⁸F, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, and ¹⁹²Ir. In certain embodiments, the radioisotope comprises ⁸⁹Zr.

In certain embodiments, the chelator is a member selected from the group consisting of deferoxamine B (DFO), 1,4,8,1 1-tetraazabicyclo[6.6.2]hexadecane-4,11- diyl)diacetic acid (CB-TE2A); diethylenetriaminepentaacetic acid (DTPA); 1,4,7, 10-tetraazacyclotetradecane-1,4,7, 10-tetraacetic acid (DOTA); thylenediaminetetraacetic acid (EDTA); ethylene glycolbis(2-aminoethyl)-N,N,N',N'- tetraacetic acid (EGTA); 1,4,8,1 1-tetraazacyclotetradecane-1,4,8,11-tetraacetic acid (TETA); ethylenebis-(2-4 hydroxy-phenylglycine) (EHPG); 5-Cl-EHPG; 5Br-EHPG; 5- Me-EHPG; 5t-Bu-EHPG; 5-sec-Bu-EHPG; benzodiethylenetriamine pentaacetic acid (benzo-DTPA); dibenzo-DTPA; phenyl-DTPA, diphenyl-DTPA; benzyl-DTPA; dibenzyl DTPA; bis-2 (hydroxybenzyl)-ethylene-diaminediacetic acid (HBED) and derivatives thereof; Ac-DOTA; benzo-DOTA; dibenzo-DOTA; 1,4,7-triazacyclononane N,N',N"- triacetic acid (NOTA); benzo-NOTA; benzo-TETA, Octadentate Hydroxypyridinonate (HOPO) ligands (e.g., 3,4,3-(LI-1,2-HOPO)), benzo-DOTMA, where DOTMA is 1,4,7, 10-tetraazacyclotetradecane-1,4,7,10-tetra(methyl tetraacetic acid), benzo-TETMA (e.g., wherein TETMA is 1,4,8,11-tetraazacyclotetradecane-1,4,8,1 1-(methyl tetraacetic acid)); derivatives of 1,3-propylenediaminetetraacetic acid (PDTA); triethylenetetraaminehexaacetic acid (TTHA); derivatives of 1,5,10-N,N',N"-tris(2,3- dihydroxybenzoyl)-tricatecholate (LICAM); and 1,3,5-N,N',N"-tris(2,3- dihydroxybenzoyl)aminomethylbenzene (MECAM), and other metal chelators. In certain embodiments, the chelator comprises deferoxamine B (DFO).

### Brief Description of the Drawings

The foregoing and other objects, aspects, features, and advantages of the present disclosure will become more apparent and better understood by referring to the following description taken in connection with the accompanying drawings, in which:

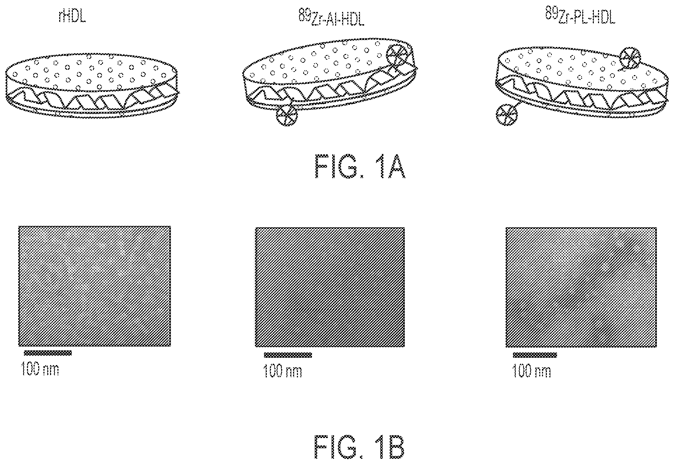

FIGS. 1A and 1B show a structure and composition of HDL and ⁸⁹Zr-HDL nanotracers.
FIG. 1A shows a schematic of HDL (left), ⁸⁹Zr-AI-HDL (middle) and ⁸⁹Zr-PL-HDL (right).
FIG. 1B shows transmission electron microscopy (TEM) images of HDL (left), ⁸⁹Zr-AI-HDL (middle) and ⁸⁹Zr-PL-HDL (right).
FIGS. 2A - 2C show size exclusion chromatograms and *in vitro* stability of ⁸⁹Zr-HDL nanoparticles.

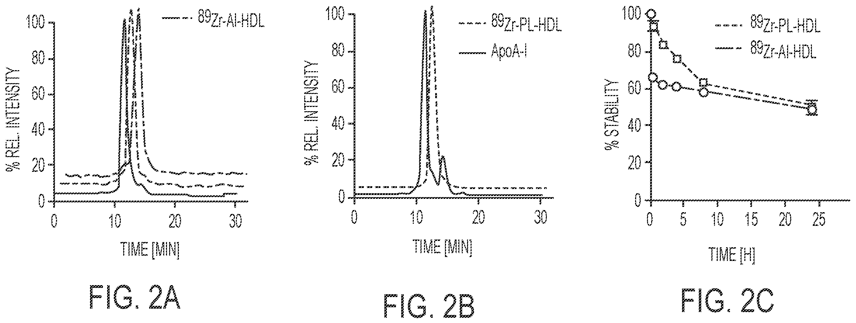

FIG. 2A shows coelution of plain HDL (black trace), DFO-ApoA-1@HDL (short dashes) and ⁸⁹Zr-AI-HDL (long and short dashes, radioactive trace).
FIG. 2B shows coelution of 1% DSPE-DFO@HDL (black trace) and ⁸⁹Zr-PL-HDL (dashes, radioactive trace).
FIG. 2C shows *in vitro* serum stability of ⁸⁹Zr-HDL nanoparticles at 37 °C.

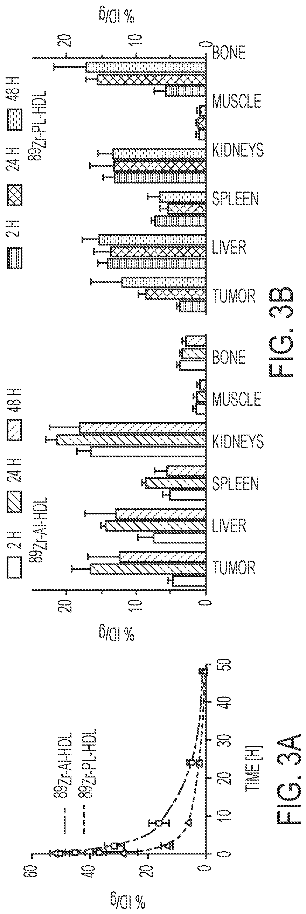

FIGS. 3A and 3B show pharmacokinetics and biodistribution of ⁸⁹Zr-HDL nanoparticles.
FIG. 3A shows a blood time-activity curve for ⁸⁹Zr-AI-HDL and ⁸⁹Zr-PL-HDL (n = 3).
FIG. 3B shows radioactivity distribution of ⁸⁹Zr-AI-HDL (left panel) and ⁸⁹Zr-PL-HDL (right panel) in selected tissues in mice bearing orthotopic breast cancer tumors.

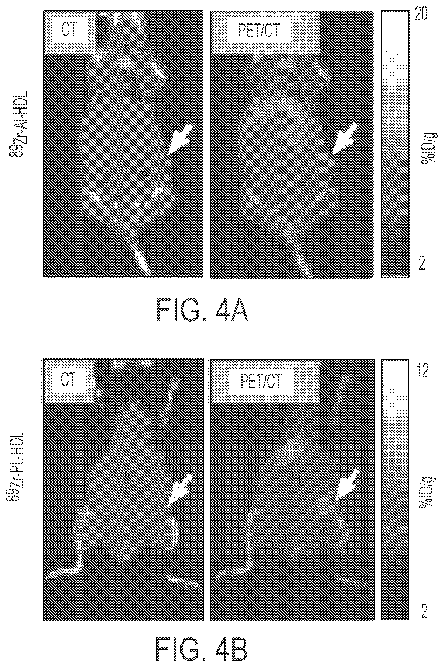

FIGS. 4A and 4B show exemplary accumulation of ⁸⁹Zr-HDL nanotracers in tumor tissues that can be visualized by *in vivo* PET imaging (CT (left) and PET/CT fusion (right)).
FIG. 4A shows images of ⁸⁹Zr-AI-HDL obtained at 24 h post injection in mice bearing orthotopic 4T1 tumors (indicated by arrows).
FIG. 4B shows images of ⁸⁹Zr-PL-HDL obtained at 24 h post injection in mice bearing orthotopic 4T1 tumors (indicated by arrows).

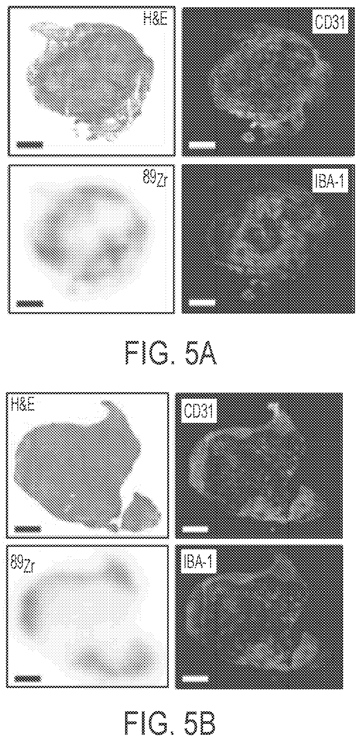

FIG. 5A shows *ex vivo* histological analysis of a tumor sections at 24 h after administration of ⁸⁹Zr-AI-HDL. Hematoxylin and eosin staining (top left), immunofluorescence for CD31 (top right) and IBA-1 (bottom right), and autoradiography (bottom left) are included. The scale bar is 2 mm.
FIG. 5B shows *ex vivo* histological analysis of a tumor sections at 24 h after administration of ⁸⁹Zr-PL-HDL. Hematoxylin and eosin staining (top left), immunofluorescence for CD31 (top right) and IBA-1 (bottom right), and autoradiography (bottom left) are included. The scale bar is 2 mm.

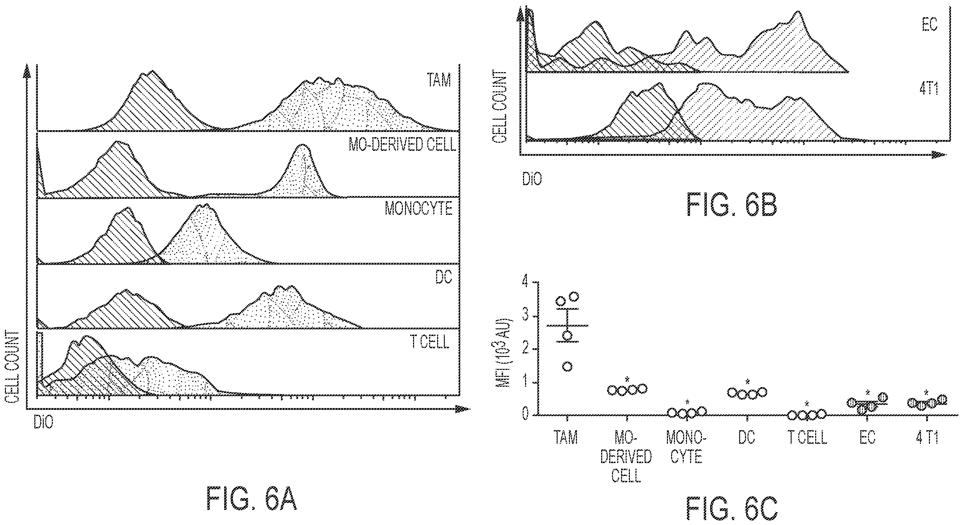

FIGS. 6A - 6C show that HDL nanoparticles preferentially target TAMs.
FIG. 6A shows representative DiO uptake in five immune cells, namely tumor-associated macrophages (TAM), monocyte-derived cells (Mo-derived cell), monocytes, dendritic cells (DC), and T cells.
FIG. 6B shows representative DiO uptake in endothelial cells (EC) and tumor cells (4T1). Cells from a PBS-injected mouse serve as controls (grey histograms to the left).
FIG. 6C shows quantification of DiO uptake presented as mean fluorescence intensity (MFI). Statistics were calculated with two-tailed Student's *t*-test with unequal variance by comparing to TAM. * *P* < 0.05.

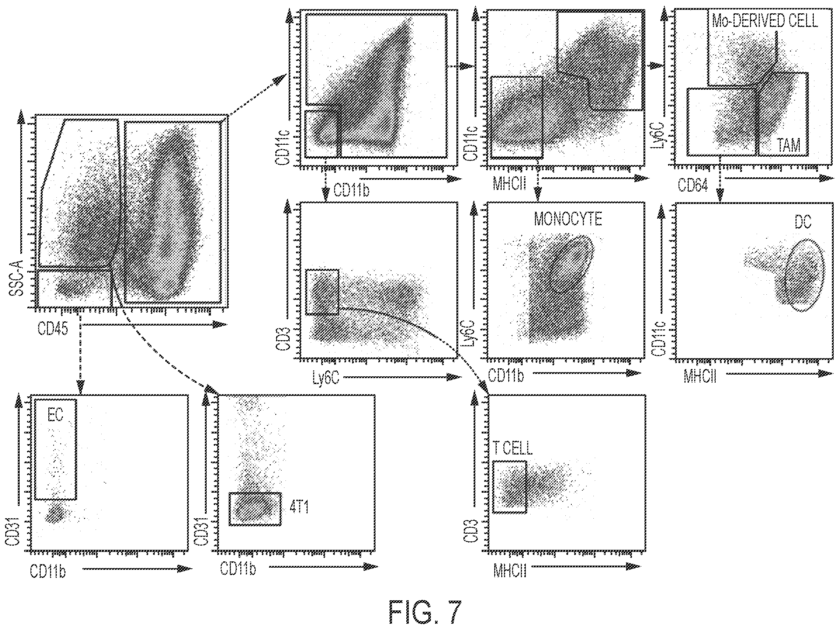

FIG. 7 shows a gating procedure to identify cells in tumor. Live single cells were subjected to the above gating procedure to identify tumor-associated macrophages (TAM), monocyte-derived cells (Mo-derived cell), monocytes, dendritic cells (DC), T cells, endothelial cells (EC), and tumor cells (4T1).

### Definitions

In order for the present disclosure to be more readily understood, certain terms are first defined below. Additional definitions for the following terms and other terms are set forth throughout the specification.

In this application, the use of "or" means "and/or" unless stated otherwise. As used in this application, the term "comprise" and variations of the term, such as "comprising" and "comprises," are not intended to exclude other additives, components, integers or steps. As used in this application, the terms "about" and "approximately" are used as equivalents. Any numerals used in this application with or without about/approximately are meant to cover any normal fluctuations appreciated by one of ordinary skill in the relevant art.

As used herein, the term "activating agent" refers to an agent whose presence or level correlates with elevated level or activity of a target, as compared with that observed absent the agent (or with the agent at a different level). In some embodiments, an activating agent is one whose presence or level correlates with a target level or activity that is comparable to or greater than a particular reference level or activity (e.g., that observed under appropriate reference conditions, such as presence of a known activating agent, e.g., a positive control).

In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

The term "administration" refers to introducing a substance into a subject. In general, any route of administration may be utilized including, for example, parenteral (e.g., intravenous), oral, topical, subcutaneous, peritoneal, intraarterial, inhalation, vaginal, rectal, nasal, introduction into the cerebrospinal fluid, or instillation into body compartments. In some embodiments, administration is oral. Additionally or alternatively, in some embodiments, administration is parenteral. In some embodiments, administration is intravenous.

The term "agent" refers to a compound or entity of any chemical class including, for example, polypeptides, nucleic acids, saccharides, lipids, small molecules, metals, or combinations thereof. As will be clear from context, in some embodiments, an agent can be or comprise a cell or organism, or a fraction, extract, or component thereof. In some embodiments, an agent is or comprises a natural product in that it is found in and/or is obtained from nature. In some embodiments, an agent is or comprises one or more entities that are man-made in that it is designed, engineered, and/or produced through action of the hand of man and/or are not found in nature. In some embodiments, an agent may be utilized in isolated or pure form; in some embodiments, an agent may be utilized in crude form. In some embodiments, potential agents are provided as collections or libraries, for example that may be screened to identify or characterize active agents within them. Some particular embodiments of agents that may be utilized include small molecules, antibodies, antibody fragments, aptamers, siRNAs, shRNAs, DNA/RNA hybrids, antisense oligonucleotides, ribozymes, peptides, peptide mimetics, peptide nucleic acids, small molecules, etc. In some embodiments, an agent is or comprises a polymer. In some embodiments, an agent contains at least one polymeric moiety. In some embodiments, an agent comprises a therapeutic, diagnostic and/or drug.

As used herein, the term "approximately" or "about," as applied to one or more values of interest, refers to a value that is similar to a stated reference value. In certain embodiments, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

As used herein, the term "associated" typically refers to two or more entities in physical proximity with one another, either directly or indirectly (e.g., via one or more additional entities that serve as a linking agent), to form a structure that is sufficiently stable so that the entities remain in physical proximity under relevant conditions, e.g., physiological conditions. In some embodiments, associated moieties are covalently linked to one another. In some embodiments, associated entities are non-covalently linked. In some embodiments, associated entities are linked to one another by specific non-covalent interactions (i.e., by interactions between interacting ligands that discriminate between their interaction partner and other entities present in the context of use, such as, for example. streptavidin/avidin interactions, antibody/antigen interactions, *etc.).* Alternatively or additionally, a sufficient number of weaker non-covalent interactions can provide sufficient stability for moieties to remain associated. Exemplary non-covalent interactions include, but are not limited to, electrostatic interactions, hydrogen bonding, affinity, metal coordination, physical adsorption, host-guest interactions, hydrophobic interactions, pi stacking interactions, van der Waals interactions, magnetic interactions, electrostatic interactions, dipole-dipole interactions, *etc.*

The term "biocompatible", as used herein is intended to describe materials that do not elicit a substantial detrimental response *in vivo.* In certain embodiments, the materials are "biocompatible" if they are not toxic to cells. In certain embodiments, materials are "biocompatible" if their addition to cells *in vitro* results in less than or equal to 20% cell death, and/or their administration *in vivo* does not induce inflammation or other such adverse effects. In certain embodiments, materials are biodegradable.

As used herein, "biodegradable" materials are those that, when introduced into cells, are broken down by cellular machinery (e.g., enzymatic degradation) or by hydrolysis into components that cells can either reuse or dispose of without significant toxic effects on the cells. In certain embodiments, components generated by breakdown of a biodegradable material do not induce inflammation and/or other adverse effects *in vivo.* In some embodiments, biodegradable materials are enzymatically broken down. Alternatively or additionally, in some embodiments, biodegradable materials are broken down by hydrolysis. In some embodiments, biodegradable polymeric materials break down into their component polymers. In some embodiments, breakdown of biodegradable materials (including, for example, biodegradable polymeric materials) includes hydrolysis of ester bonds. In some embodiments, breakdown of materials (including, for example, biodegradable polymeric materials) includes cleavage of urethane linkages.

As used herein, "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E. W. Martin.

As used herein, the term "combination therapy", refers to those situations in which two or more different pharmaceutical agents for the treatment of disease are administered in overlapping regimens so that the subject is simultaneously exposed to at least two agents. In some embodiments, the different agents are administered simultaneously. In some embodiments, the administration of one agent overlaps the administration of at least one other agent. In some embodiments, the different agents are administered sequentially such that the agents have simultaneous biologically activity with in a subject.

As used herein, the term "excipient" refers to a non-therapeutic agent that may be included in a pharmaceutical composition, for example to provide or contribute to a desired consistency or stabilizing effect. Suitable pharmaceutical excipients include, for example, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

As used herein, a "functional" biological molecule is a biological molecule in a form in which it exhibits a property and/or activity by which it is characterized. A biological molecule may have two functions (i.e., bifunctional) or many functions (i.e., multifunctional).

The term *"in vitro"* as used herein refers to events that occur in an artificial environment, e.g., in a test tube or reaction vessel, in cell culture, etc., rather than within a multi-cellular organism.

As used herein *"in vivo"* refers to events that occur within a multi-cellular organism, such as a human and a non-human animal. In the context of cell-based systems, the term may be used to refer to events that occur within a living cell (as opposed to, for example, *in vitro* systems).

The term "imaging agent" as used herein refers to any element, molecule, functional group, compound, fragments thereof or moiety that facilitates detection of an agent (e.g., a polysaccharide nanoparticle) to which it is joined. Examples of imaging agents include, but are not limited to: various ligands, radionuclides (e.g., ³H, ¹⁴C, ¹⁸F, ¹⁹F, ³²P, ³⁵S, ¹³⁵I, ¹²⁵I, ¹²³I, ⁶⁴Cu, ¹⁸⁷Re, ¹¹¹In, ⁹⁰Y, ^{99m}Tc, ¹⁷⁷Lu, ⁸⁹Zr etc.), fluorescent dyes (for specific exemplary fluorescent dyes, see below), chemiluminescent agents (such as, for example, acridinum esters, stabilized dioxetanes, and the like), bioluminescent agents, spectrally resolvable inorganic fluorescent semiconductors nanocrystals (i.e., quantum dots), metal nanoparticles (e.g., gold, silver, copper, platinum, etc.) nanoclusters, paramagnetic metal ions, enzymes (for specific examples of enzymes, see below), colorimetric labels (such as, for example, dyes, colloidal gold, and the like), biotin, dioxigenin, haptens, and proteins for which antisera or monoclonal antibodies are available.

As used herein, the term "nanoparticle" refers to a particle having a diameter of less than 1000 nanometers (nm). In some embodiments, a nanoparticle has a diameter of less than 300 nm, as defined by the National Science Foundation. In some embodiments, a nanoparticle has a diameter of less than 100 nm as defined by the National Institutes of Health. In some embodiments, nanoparticles are micelles in that they comprise an enclosed compartment, separated from the bulk solution by a micellar membrane, typically comprised of amphiphilic entities which surround and enclose a space or compartment (e.g., to define a lumen). In some embodiments, a micellar membrane is comprised of at least one polymer, such as for example a biocompatible and/or biodegradable polymer.

As used herein, the term "substantially", and grammatical equivalents, refers to the qualitative condition of exhibiting total or near-total extent or degree of a characteristic or property of interest. One of ordinary skill in the art will understand that biological and chemical phenomena rarely, if ever, go to completion and/or proceed to completeness or achieve or avoid an absolute result.

As used herein, the term "subject" includes humans and mammals (e.g., mice, rats, pigs, cats, dogs, and horses). In many embodiments, subjects are mammals, particularly primates, especially humans. In some embodiments, subjects are livestock such as cattle, sheep, goats, cows, swine, and the like; poultry such as chickens, ducks, geese, turkeys, and the like; and domesticated animals particularly pets such as dogs and cats. In some embodiments (e.g., particularly in research contexts) subject mammals will be , for example, rodents (e.g., mice, rats, hamsters), rabbits, primates, or swine such as inbred pigs and the like.

As used herein, the term "treatment" (also "treat" or "treating") refers to any administration of a substance that partially or completely alleviates, ameliorates, relives, inhibits, delays onset of, reduces severity of, and/or reduces incidence of one or more symptoms, features, and/or causes of a particular disease, disorder, and/or condition. Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition. In some embodiments, treatment may be of a subject who has been diagnosed as suffering from the relevant disease, disorder, and/or condition. In some embodiments, treatment may be of a subject known to have one or more susceptibility factors that are statistically correlated with increased risk of development of the relevant disease, disorder, and/or condition.

### Detailed Description

The invention is defined by the claims. Any subject-matter falling outside the scope of the claims is provided for information purposes only.

Throughout the description, where compositions, articles, and devices are described as having, including, or comprising specific components, or where processes and methods are described as having, including, or comprising specific steps, it is contemplated that, additionally, there are compositions, articles, and devices of the present invention that consist essentially of, or consist of, the recited components, and that there are processes and methods according to the present invention that consist essentially of, or consist of, the recited processing steps.

Similarly, where compositions, articles, and devices are described as having, including, or comprising specific compounds and/or materials, it is contemplated that, additionally, there are compositions, articles, and devices of the present invention that consist essentially of, or consist of, the recited compounds and/or materials.

Described herein are natural macrophage-targeting principles and TAM-specific radiolabeled bio-nanoparticles based on high-density lipoproteins (HDLs). A lipoprotein is a natural particle composed of proteins and lipids. A lipoprotein helps fats to move through the water inside and outside cells, emulsifying the lipid molecules. A high-density lipoprotein (HDL) is a biological nanoparticle that transports fats within the body and has an important role in reverse cholesterol transport system. There are several subclasses of HDL that differ in lipid and protein (apolipoprotein) composition. The main apolipoprotein found in HDL is Apolipoprotein 1 (ApoAl), through which it binds to several membrane receptors abundantly expressed on macrophages. HDL can be used for atherosclerosis magnetic resonance molecular imaging of plaque macrophages, or to deliver an anti-inflammatory drug to atherosclerotic lesions with great specificity. An HDL's density is usually greater than 1.063 g/ml (e.g., measured by ultracentrifugation combined with electrophoresis).

Also described herein (e.g., to enable HDL's use for quantitative PET imaging of TAM) is a design, synthesis, and pharmacokinetic evaluation of two different ⁸⁹Zr-modified HDL (HDL) nanoparticles. Specifically, ApoAl or HDL's phospholipid load were labeled, and the agent's ⁸⁹Zr-TAM targeting was examined using *in vivo* PET imaging and *ex vivo* analyses, including immunohistochemistry. Additionally, a fluorescent surrogate marker was prepared for the radiolabeled ⁸⁹Zr-HDL particles, which was used to investigate the intercellular distribution of this particle class by flow cytometry. When intravenously injected in an orthotopic mouse model of cancer, the prime tumor target of this surrogate marker are TAMs, followed by monocyte-derived cells and dendritic cells.

Described herein are specific and quantifiable TAM imaging agents for use in evaluating the efficacy of TAM-targeting therapies and facilitating prognosis of TAM-driven cancers. The present embodiments provide, among other things, certain radioisotope labeled lipoproteins, their uses and synthesis methods.

Certain provided compositions such as radiolabeled HDL nanoparticles may be prepared via incorporation of a long-lived positron-emitting nuclide (e.g., radioisotope) ⁸⁹Zr into a lipoprotein (HDL) comprising phospholipids and apolipoproteins. In some embodiments, nanoparticles are composed of the phospholipid DMPC and apolipoprotein 1 (ApoAl) in a 2.5:1 weight ratio. In some embodiments, a positron-emitting nuclide may be complexed with a chelator conjugated to either a phospholipid or apolipoproteins. In some embodiments, ⁸⁹Zr is complexed with DFO, conjugated to either a phospholipid or ApoA1 protein to generate ⁸⁹Zr-PL-HDL and ⁸⁹Zr-AI-HDL, respectively. In some experimental examples presented herein, *in vivo* evaluation was carried out in orthotopic mouse models of breast cancer and included pharmacokinetic analysis, biodistribution studies, and PET imaging. *Ex vivo* histological analysis of tumor tissues to assess regional distribution of ⁸⁹Zr radioactivity was also performed. Fluorescent analogs of the radiolabeled agents were used to determine cell specificity via flow cytometry.

In some embodiments, HDL-comprising phospholipids comprise dimiristoyl phosphatidyl choline (DMPC), 1,2-dipentadecanoyl-sn-glycero-3-phosphocholine, 1,2-dipalmitoyl-sn-glycero-3-phosphocholine (DPPC), 1,2-diheptadecanoyl-sn-glycero-3-phosphocholine, 1,2-distearoyl-sn-glycero-3-phosphocholine (DSPC), 1-myristoyl-2-hydroxy-sn-glycero-3-phosphocholine (MHPC), 1-pentadecanoyl-2-hydroxy-sn-glycero-3-phosphocholine, 1-palmitoyl-2-hydroxy-sn-glycero-3-phosphocholine (PHPC), 1-heptadecanoyl-2-hydroxy-sn-glycero-3-phosphocholine, 1-stearoyl-2-hydroxy-sn-glycero-3-phosphocholine (SHPC), 1-oleoyl-2-hydroxy-sn-glycero-3-phosphocholine (OHPC), 1,2-dioleoyl-sn-glycero-3-phosphocholine (DOPC), and/or 1,2-dipalmitoleoyl-sn-glycero-3-phosphocholine. As used herein, the term "phospholipid" includes phospholipid derivatives (including natural and synthetic phospholipid derivatives). In some embodiments, HDL-comprising apolipoproteins comprise Apolipoprotein 1 (ApoA-1). In some embodiments, a weight ratio of phospholipid and protein may be about 2:1 to about 3:1, about 2.2:1 to about 2.8:1, about 2.3:1 to about 2.7:1, or about 2.4:1 to about 2.6:1.

In some embodiments, HDL nanoparticles may be radiolabeled via incorporation of chelator-modified apolipoproteins with radioisotopes and/or incorporation of chelator-modified phospholipids with radioisotopes. In some embodiments, chelator-modified apolipoproteins comprise ApoA1- deferoxamine B (DFO). In some embodiments, chelator-modified phospholipids comprise DMPC-DFO and DSPE-DFO.

In some embodiments, discoidal HDL nanoparticles may be synthesized by
(i) modifying one or more apolipoproteins with a chelator and reacting the modified apolipoproteins with a radioisotope comprising a moiety reactive with the chelator, and incorporating the radiolabeled chelator-modified apolipoproteins in the nanoparticle,
(ii) modifying one or more apolipoproteins with a chelator, incorporating the chelator-modified apolipoproteins in the nanoparticle, and reacting the chelator-modified apolipoproteins with a radioisotope comprising a moiety reactive with the chelator;
(iii) modifying one or more phospholipids with a chelator, reacting the modified phospholipids with a radioisotope comprising a moiety reactive with the chelator, and incorporating the radiolabeled chelator-modified phospolipids in the nanoparticle; or
(iv) modifying one or more phospholipids, incorporating the chelator-modified phospholipids in the nanoparticle, and reacting the chelator-modified phospholipids with a radioisotope comprising a moiety reactive with the chelator.

In some embodiments, radioisotopes comprise ^{99m}Tc, ¹¹¹In, ⁶⁴Cu, ⁶⁷Ga, ¹⁸⁶Re, ¹⁸⁸Re, ¹⁵³Sm, ¹⁷⁷Lu, ⁶⁷Cu, ¹²³I, ¹²⁴I, ¹²⁵I, ¹¹C, ¹³N, ¹⁵O, ¹⁸F, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, ⁸⁹Zr, and ¹⁹²Ir.

In certain embodiments, the nanoparticle comprises a chelator, for example, 1,4,8,11-tetraazabicyclo[6.6.2]hexadecane-4,11- diyl)diacetic acid (CB-TE2A); desferoxamine (DFO); diethylenetriaminepentaacetic acid (DTPA); 1,4,7, 10-tetraazacyclotetradecane- 1,4,7, 10-tetraacetic acid (DOTA); thylenediaminetetraacetic acid (EDTA); ethylene glycolbis(2-aminoethyl)-N,N,N',N'- tetraacetic acid (EGTA); 1,4,8,11-tetraazacyclotetradecane-1,4,8,11-tetraacetic acid (TETA); ethylenebis-(2-4 hydroxy-phenylglycine) (EHPG); 5-Cl-EHPG; 5Br-EHPG; 5- Me-EHPG; 5t-Bu-EHPG; 5-sec-Bu-EHPG; benzodiethylenetriamine pentaacetic acid (benzo-DTPA); dibenzo-DTPA; phenyl-DTPA, diphenyl-DTPA; benzyl-DTPA; dibenzyl DTPA; bis-2 (hydroxybenzyl)-ethylene-diaminediacetic acid (HBED) and derivatives thereof; Ac-DOTA; benzo-DOTA; dibenzo-DOTA; 1,4,7-triazacyclononane N,N',N"- triacetic acid (NOTA); benzo-NOTA; benzo-TETA, benzo-DOTMA, where DOTMA is 1,4,7, 10-tetraazacyclotetradecane-1,4,7,10-tetra(methyl tetraacetic acid), benzo-TETMA, where TETMA is 1,4,8,11-tetraazacyclotetradecane-1,4,8,11-(methyl tetraacetic acid); derivatives of 1,3-propylenediaminetetraacetic acid (PDTA); triethylenetetraaminehexaacetic acid (TTHA); derivatives of 1,5,10-N,N',N"-tris(2,3-dihydroxybenzoyl)-tricatecholate (LICAM); and 1,3,5-N,N',N"-tris(2,3- dihydroxybenzoyl)aminomethylbenzene (MECAM), or other metal chelators.

In some embodiments, incorporation of radioisotopes may not have measurable effect on size compared to HDL without radioisotopes. In some embodiments, radiolabeling of modified chelators may be stable to perform *in vivo* visualization, having permanent association of radioisotopes to a nanoparticle fraction.

In some embodiments, radiolabeled nanoparticles may be visualized *in vivo* with PET/CT. In some embodiments, radiolabeled nanoparticles may include fluorophore (e.g., near-infrared fluorophore) visualized *in vivo* by PET and near-infrared fluorescence imagine.

In some embodiments, HDL nanoparticles may have molecular weight within a range from about 100 kDa to about 400 kDa, from about 125 kDa to about 375 kDa, or from about 150 kDa to about 350 kDa as measured by size exclusion chromatography.

In some embodiments, HDL nanoparticles may have average diameter within a range from about 5 nm to about 40 nm, from about 5 nm to 30 nm, or from about 5 nm to 20 nm, as measured by Dynamic Light Scattering.

In some embodiments, blood residence time of HDL nanoparticles (e.g., in mice) may be from about 1 hour to about 20 hours, from about 1 hour to about 15 hours, from about 1 hour to about 10 hours, from about 1 hour to about 8 hours, from about 1 hour to about 6 hours, from about 2 hours to about 6 hours, from about 2 hours to about 8 hours, from about 2 hours to about 10 hours, from about 2 hours to about 15 hours, or from about 2 hours to about 20 hours. Without wishing to be bound by any particular theory, blood residence time may reflect behavior of components in natural HDL. For example, phospholipids transported by HDL exchange with other lipoproteins before they are ultimately cleared from circulation when delivered to their targets. HDL nanoparticles with a low net internalization and/or catabolic rate may have long circulation times.

In some embodiments, tumor (e.g., mouse breast cancer) uptake of HDL nanoparticles may peak from about 12 hours to about 60 hours, from about 18 hours to about 54 hours, from about 21 hours to about 51 hours, or from about 24 hours to about 48 hours after injection.

HDL nanoparticles may be detected in organs. Without wishing to be bound by any particular theory, accumulation of HDL nanoparticles may be observed where organ process catabolism. Accumulation of activity in the mineral component of bone may be the result of liberation of radioisotope from its chelator.

TAMs may be imaged with high-density lipoprotein positron emission tomography (HDL PET). In some embodiments, TAMs may be imaged by administering to a subject a composition comprising the discoidal high-density lipoprotein nanoparticle, allow the nanoparticle to accumulate in a region of TAMs (e.g., by waiting a period of time) and detecting the radioisotope via PET after accumulation of the nanoparticle in the region of TAM.

Imaging compositions incorporating HDL nanoparticles described herein may be administered according to any appropriate route and regimen. Compositions described herein may be administered by any route, as will be appreciated by those skilled in the art. In some embodiments, compositions described herein are administered by oral (PO), intravenous (IV), intramuscular (IM), intra-arterial, intramedullary, intrathecal, subcutaneous (SQ), intraventricular, transdermal, interdermal, intradermal, rectal (PR), vaginal, intraperitoneal (IP), intragastric (IG), topical (*e.g.,* by powders, ointments, creams, gels, lotions, and/or drops), mucosal, intranasal, buccal, enteral, vitreal, sublingual; by intratracheal instillation, bronchial instillation, and/or inhalation; as an oral spray, nasal spray, and/or aerosol, and/or through a portal vein catheter.

### Experimental Examples

### Example 1: Radiolabeling HDL nanoparticles

This example demonstrates exemplary HDL labeling approaches, including: (1) attachment of the radiolabel to Apo-AI, the main apolipoprotein component of HDL; and (2) radiolabeling of the phospholipid load of the particle as shown in FIGS. 1A and 1B. ⁸⁹Zr was selected as the radioisotope for this example, as its physical half-life (78.2 h) matches the long biological half-life of HDL.

### Chemicals:

Phospholipids were purchased from Avanti Polar Lipids, and 1-(4-Isothiocyanatophenyl)-3-[6,17-dihyroxy-7,10,18,21-tetraoxo-27-[N-acetylhydroxylamino) 6,11,17, 22 -tetraazaheptaeicosane]thiourea (DFO-p-NCS) by Macrocyclics. The dyes 3,3'-dioctadecyloxacarbocyanine perchlorate (DiO) and Cyanine5 NHS ester were purchased from Life Technologies and Lumiprobe, respectively. ApoAl was separated from human plasma using an established protocol. Antibodies for flow cytometry were purchased from eBiosciences, Biolegend, and BD Bioscience. All other reagents were acquired from Sigma-Aldrich.

### Synthesizing the phospholipid-chelator DSPE-DFO:

1,2-distearoyl-sn-glycero-3-phosphoethanolamine (DSPE) and 1-(4-Isothiocyanatophenyl)-3-[6,17-dihyroxy-7,10,18,21-tetraoxo-27-[N-acetylhydro-xylamino)-6,11,17,22-tetraaza-heptaeicosane]thiourea (DFO-p-NCS) were reacted in a 1:1 dimethylsulfoxide/chloroform mixture in the presence of diethyl isopropylaimne at 50 °C for 48 h under a nitrogen atmosphere. After cooling down to room temperature, chloroform was evaporated and water was added along with a 1 M Tris solution. The mixture was stirred for 30 min and filtered. The solid was washed with 1 M Tris, water and dichloromethane to produce the desired compound as a white solid in 70-80 % yield.

### HDL preparation:

HDL was prepared by mixing 1,2-dimyristoyl-sn-glycero-3-phosphocholine (DMPC) and ApoAl in a 2.5:1 weight ratio. A lipid film was formed by evaporation of a chloroform solution containing the phospholipid. Hydration with PBS at 35 - 40 °C and sonication was followed by the addition of the required amount of ApoAl. Sonication on ice for 10 minutes yielded a slightly turbid solution that was kept at 37 °C overnight. Subsequent centrifugation at 4000 rpm for 5 min and filtration through 0.22 µm filter afforded a clear solution of HDL. For the preparation of 1 % DSPE-DFO@HDL, the required amount of the phospholipid-chelator DSPE-DFO (1) was added at the expense of DMPC and the same procedure was followed. This resulted in discoidal particles with a mean hydrodynamic diameter of 8.7 ± 0.9 nm (n=6), as measured by DLS.

### Modification of HDL with desferrioxamine B:

DFO-*p*-NCS (dissolved in DMSO, 5 mg/mL) was added in steps of 5 µL to a solution of HDL in 0.1 M PBS buffer pH 8.2, typically containing 2 mg of ApoA1 per mL. The mixture was vortexed after each addition until a 2-fold molar excess of DFO-*p*-NCS over ApoAl was achieved, and then incubated for 2 hours at 37 °C. The particles were separated from free, unreacted DFO-*p*-NCS by spin filtration using 10 kDa molecular weight cut-off (MWCO) Vivaspin 500 (Sartorius Stedim Biotech Gmbh, Goettingen, Germany) tubes at 15000 rpm. The concentrate was washed 4 times with 500 µL PBS pH 7.4 and diluted to the final volume with PBS. The number of labels per ApoA1 molecule was measured to be 1.4 ± 0.3 (n=3) by the isotope dilution method. The resulting DFO-ApoA1@HDL had a diameter of 8.9 ± 1.1 nm (n=5).

### Radiochemistry:

⁸⁹Zr was produced at Memorial Sloan-Kettering Cancer Center on an EBCO TR19/9 variable-beam energy cyclotron (Ebco Industries Inc., British Columbia, Canada) via the ⁸⁹Y(p,n)⁸⁹Zr reaction and purified in accordance with previously reported methods to yield ⁸⁹Zr with a specific activity of 195-497 MBq/µg. Activity measurements were made using a Capintec CRC-15R Dose Calibrator (Capintec, Ramsey, NJ).

### Radiolabling AproA-I at HDL (⁸⁹Zr-AI-HDL):

A solution of ⁸⁹Zr in PBS was prepared by mixing 100 uL PBS with the corresponding volume of ⁸⁹Zr-oxalate solution in 1M oxalic acid and adjusted to pH 7.1 - 7.4 with 1M Na₂CO₃. Labeling HDL precursor DFO-ApoA1 in HDL was then added as a PBS solution containing 2 mg ApoA1/mL. The labeling mixture was prepared at an activity-to-ApoAl ratio of 1 mCi/mg ApoA1, and incubated at 37 °C for 2 h. Subsequent isolation by spin-filtration using 10 kDa MWCO Vivaspin 500 tubes and washing with PBS (4x500 uL) afforded radiochemically pure ⁸⁹Zr-AL-HDL. The retentate was diluted with PBS and filtered through a 0.22 µm filter prior to use.

### Radiolabling phospolipid at HDL (⁸⁹Zr-PL-HDL):

⁸⁹Zr-PL-HDL was obtained following the same procedure described above, using the corresponding labeling precursor 1 % DSPE-DFO@HDL. 1 % DSPE-DFO@HDL with a mean diameter of 8.6 ± 1.3 nm (n=5) was obtained.

Modifications had no measurable effect on the size (measured by NanoSeries Z-Sizer (Malvern Instruments, Malvern, UK)) compared to plain HDL (Table 1). In both cases (e.g., ⁸⁹Zr-AI-HDL and ⁸⁹Zr-PL-HDL), the radiolabeling of the modified precursors resulted in the permanent association of the radioisotope to a particle fraction of an estimated molecular weight of 150 kDa. This molecular weight is in concordance with the expected molecular weight of discoidal HDL.

Table 1 below illustrates composition (in mol %), size and surface charge of HDL, ⁸⁹Zr-AI-HDL and ⁸⁹Zr-PL-HDL.

**Table 1**

| | ApoA1 | DMPC | DSPE-DFO | Size (nm) | Z-Potential (mV) |
|---|---|---|---|---|---|
| HDL | 1% | 99% | - | 8.7 ± 0.9 | -14.6 ± 3.2 |
| ⁸⁹Zr-AI-HDL | 1% | 99% | - | 8.5 ± 1.1 | -16.2 ± 1.6 |
| ⁸⁹Zr-PL-HDL | 1% | 98% | 1% | 8.5 ± 0.8 | -13.5 ± 6.6 |

### Fluorescent HDL nanoparticles:

Phospholipids (DMPC) and DiO were mixed in chloroform solution at a weight ratio of 99:1. A thin film was formed by evaporating the solvent, and large vesicles were made by hydrating the film with apoA-1 solution. Small-sized particles were created by an ultrasonic sonication procedure and big aggregates were removed by centrifuge and filtration.

### HPLC and Radio-HPLC:

HPLC was performed on a Shimadzu HPLC system equipped with two LC-10AT pumps and an SPD-M10AVP photodiode array detector. Radio-HPLC was performed using a Lablogic Scan-RAM Radio-TLC/HPLC detector. Size exclusion chromatography was performed on a Superdex 10/300 column (GE Healthcare Life Sciences, Pittsburg, PA) using PBS as eluent at a flow rate of 1 mL/min.

### Preparation for Transmission electron microscopy (TEM):

The original PBS-based solvent of nanoparticles was replaced with an ammonium acetate buffer. Then, the particles were mixed with a 2% sodium phosphotungstate (pH = 7.4) buffer to achieve negative stain. The mixed solution was added to TEM grids, dried, and imaged with a Hitachi H7650 system linked to a Scientific Instruments and Applications digital camera controlled by the Maxim CCD software. One-hundred-thousand-fold magnification was used to capture the images.

### Example 2: In vitro serum stability of radiolabeled HDL nanoparticles

This example demonstrates an exemplary *in vitro* stability test of ⁸⁹Zr-labeled HDL nanoparticles in serum as shown in FIGS. 2A - 2C, showing that the nanoparticles are stable to be tested *in vivo.*

A sample of the corresponding radiolabeled HDL preparation (typically 1.5-2.0 MBq in 40-60 µL PBS) was added to 400 µL of FBS. The mixture was incubated at 37 °C for 24 h. Aliquots of 0.3-0.4 MBq were taken at predetermined time points (30 min, 2 h, 4 h, 8 h and 24 h) for size exclusion chromatography analysis by careful integration of the peaks.

For ⁸⁹Zr-AI-HDL, a new peak eluting at the same retention time as free ApoAl was detected. The ratio between ⁸⁹Zr-AI-HDL and this species remained largely constant over time (Table 2). Another species having a molecular weight greater than 300 kDa was observed at all time points. ⁸⁹Zr-PL-HDL showed a similar dynamic behavior and a peak corresponding to larger particles having a molecular weight greater than 300 kDa was also observed at all time points. Interestingly, activity directly associated with albumin was not detectable until 8 h and, in any case, the majority of it (63.3 ± 1.5 %) remained bound to HDL particles (FIG. 1C). The release of small radiolabeled species was detectable after 24 h for ⁸⁹Zr-AI-HDL (5.5 ± 0.7 %, n=3) and after 2 h for ⁸⁹Zr-PL-HDL (3.3 ± 0.6 %, n=3, then reached 11.7 ± 6.4 %, n = 3, after 24 h). This could be due to release of ⁸⁹Zr from its DFO complex or a result of the degradation of the thiourea bond in the presence of oxidizing chlorinated species (19) resulting in the detachment of the ⁸⁹Zr-DFO unit. Collectively, this data suggest that both ⁸⁹Zr-AI-HDL and ⁸⁹Zr-PL-HDL are sufficiently stable to allow adequate *in vivo* evaluation.

The blood residence time differences reflect the different behavior of both components in natural HDL. The protein-labeled ⁸⁹Zr-AI-HDL showed a significantly longer blood half-life (5.7 h) as opposed to the 2.0 h half-life observed for ⁸⁹Zr-PL-HDL. It is well known that phospholipids transported by HDL exchange with other lipoproteins before they are ultimately cleared from circulation when delivered to their targets. On the other hand, the net internalization and catabolic rate of ApoAl are very low, thus lengthening its circulation time, compared to the phospholipid-labeled nanoparticle. As a result, the ⁸⁹Zr-AI-HDL-associated radioactivity half-life seems to match the slow turnover of HDL in the organism.

### Example 3: Pharmacokinetics and biodistributions of radiolabeled HDL nanoparticles

This example demonstrates exemplary pharmacokinetics and biodistribution of radiolabeled HDL nanoparticles, indicating that nanoparticles may not only be delivered passively but also actively distributed, and governed by its natural biological function.

### Cell Culture:

The mouse breast cancer cell line 4T1 was obtained from ATCC (Manassas, VA) and grown in Dulbecco's Modified Eagle's Medium (DMEM) with 4.5 g/L L-glucose, 10% (vol/vol) heat inactivated fetal bovine serum, 100 IU penicillin, and 100 µg/mL streptomycin and purchased from the culture media preparation facility at Memorial Sloan Kettering Cancer Center (MSKCC New York, NY).

### Animals:

Female homozygous athymic nude NCr mice were obtained from Taconic Laboratories (Hudson, NY), whereas female C57BL/6 (B6) mice were purchased from Charles Rivers Laboratories (Wilmington, MA). For orthotopic injections, mice were anesthetized with a 150 mg/kg ketamine and 15 mg/kg xylazine cocktail (10 µL) and an incision was made above the mammary fat pad after sterilization of the region. Then, 4T1 cells (1×10⁶ cells in 100 µL DMEM) were injected into the mammary fat pad, before the incision was sealed (Vetbond, 3M, St. Paul, MN) and the tumors grown for 8 days. For all intravenous injections, mice were gently warmed with a heat lamp, placed on a restrainer, tail sterilized with alcohol pads, and the injection was placed into the lateral tail vein.

### Pharmacokinetics experiments:

Healthy female NCr mice (8-10 weeks old, n = 6) were injected with 1.22 ± 0.02 MBq (range 1.21-1.26 MBq; 30-40 µg ApoAl) of ⁸⁹Zr-HDL preparation in 200 µL PBS solution. Blood was sampled from the saphenous vein at predetermined time points (5 min, 30 min, 2 h, 8 h, 24 h and 48 h) and radioactivity measured on a Wizard² 2470 Automatic Gamma Counter (Perkin Elmer, Waltham, MA). Measurements were carried out in triplicate and the radioactivity content was calculated as the mean percentage injected dose per gram of tissue (%ID/g) ± S.D. The weighted half-life (t_{1/2}) value measured for ⁸⁹Zr-AI-HDL was 5.7 h, nearly 3 times longer than that shown by ⁸⁹Zr-PL-HDL, whose t_{1/2} was 2.0 h as shown in FIG. 3A.

### Biodistribution experiments:

Biodistribution experiments were conducted on female Black 6 mice (6-10 weeks old, n = 20) bearing orthotopic 4T1 breast tumors. The radiolabeled HDL preparation (1.35 ± 0.05 MBq, range 1.28-1.42 MBq; 35-40 µg ApoA1, in 200 µL PBS solution) was administered via the lateral tail vein, and allowed to circulate for various time points (2 h, 24 h and 48 h), after which the mice were sacrificed and the organs perfused. The radioactive content in tissues of interest (blood, tumor, large and small intestines, stomach, kidneys, brain, bone, liver, lungs, heart, skin, spleen, bladder, tail) was measured using a 2470 Wizard2 Automatic Gamma Counter (Perkin Elmer, Waltham, MA) and the tissue associated activity was calculated as the mean percentage injected dose per gram of tissue (%ID/g) ± S.D.

A selection of tissues is shown in FIG. 3B for both formulations, and a complete list of values can be found in Tables 2 and 3 below. Most of the radioactivity remains in blood at 2 h post-injection (p.i.). Significant accumulation was also observed in kidneys (16.4 ± 2.1 %ID/g [⁸⁹Zr-AI-HDL] and 13.1 ± %1.6 ID/g [⁸⁹Zr-PL-HDL]), liver (significantly higher for ⁸⁹Zr-PL-HDL [14.1 ± 1.5 %ID/g] than ⁸⁹Zr-AI-HDL [7.51 ± 2.21 %ID/g]), and, to a lesser extent, spleen (5.0 ± 1.2 %ID/g and 7.2 ± 0.4 %ID/g, respectively). Tumor uptake at this time point is below 5 % for both tracers.

At 24 h p.i., blood activity levels had dropped to 5.30 ± 0.94 and 2.19 ± 0.23 %ID/g for ⁸⁹Zr-AI-HDL and ⁸⁹Zr-PL-HDL, respectively. A dramatic increase in tumor uptake can be observed for ⁸⁹Zr-AI-HDL, reaching 16.5 ± 2.8 %ID/g, whereas that of ⁸⁹Zr-PL-HDL was 8.6 ± 1.3 %ID/g. Kidney uptake was high for both nanotracers, but it was significantly higher for ⁸⁹Zr-AI-HDL at 21.2 ± 1.9 %ID/g. Of note, whole bone activity for ⁸⁹Zr-PL-HDL went up to 15.5 ± 1.9 %ID/g at this time point. Liver and spleen still retained a significant amount of activity.

By 48 h p.i., less than 1 %ID/g remained in circulation (0.98 ± 0.25 vs 0.49 ± 0.06 %ID/g for ⁸⁹Zr-AI-HDL and ⁸⁹Zr-PL-HDL, respectively). Liver, spleen, and kidney uptake are statistically identical for both radiolabeling approaches. Similarly, tumor uptake was 12.3 ± 4.5 %ID/g for ⁸⁹Zr-AI-HDL and 12.0 ± 4.7 %ID/g for ⁸⁹Zr-PL-HDL. The biggest discrepancy in biodistribution profiles at 48 h remained bone uptake, which was 17.1 ± 4.8 %ID/g for ⁸⁹Zr-PL-HDL and remained at 2.70 ± 0.62 %ID/g for ⁸⁹Zr-AI-HDL. 3.6 ± 1.1 % of the uptake (⁸⁹Zr-PL-HDL) originates from bone marrow, leaving 96.4 ± 1.1 % associated with mineral bone, whereas for ⁸⁹Zr-AI-HDL the fraction originating from bone marrow was 27.8 ± 4.1 %.

The high accumulation of radioactivity was observed in the kidney, especially for ⁸⁹Zr-AI-HDL. Kidneys play an important role in ApoA1 catabolism, where it is taken up and degraded (27). Without wishing to be bound by any particular theory, ApoA1 may be filtered through the glomerular basement membranes, followed by proximal tubule uptake, which enables endocytosis of HDL proteins (28). In fact, a higher glomerular filtration rate is associated with low HDL and ApoA1 levels in humans (29).

Tumor uptake was high for both formulations, peaking at 16.5 ± 2.8 %ID/g at 24 h p.i. for ⁸⁹Zr-AI-HDL, and 12.0 ± 4.7 %ID/g at 48 h p.i for ⁸⁹Zr-PL-HDL.

High accumulation of radioactivity in the bones of mice injected with ⁸⁹Zr-PL-HDL was observed. For ⁸⁹Zr-AI-HDL, whole bone uptake remained below 4 %ID/g at all time points, but a progressive increase is observed for ⁸⁹Zr-PL-HDL, reaching 17 %ID/g at 48 h p.i. This was mainly associated with mineral bone for both probes, as less than 5 and 30 % of whole bone activity was taken up by bone marrow for ⁸⁹Zr-PL-HDL and ⁸⁹Zr-AI-HDL, respectively. The high accumulation of activity in the mineral component of the bone has been reported for other long-circulating ⁸⁹Zr-labeled agents (30,31), seemingly resulting from liberation of ⁸⁹Zr from its chelator (32). These data were largely in agreement with the respective PET imaging signatures as shown in Example 4.

Table 2 below illustrates tissue radioactivity distribution of ⁸⁹Zr-AI-HDL in female C57BL/6 (B6) mice bearing orthotopic 4T1 breast cancer tumors. Data presented as mean %ID/g ± SD (n ≥ 3 for each time point).

**Table 2**

| Tissue | 2 h | 24 h | 48 h |
|---|---|---|---|
| Blood | 34.5 ± 6.9 | 5.30 ± 0.94 | 0.98 ± 0.25 |
| Tumor | 4.53 ± 0.76 | 16.5 ± 2.8 | 12.3 ± 4.5 |
| Heart | 2.67 ± 0.81 | 3.05 ± 0.17 | 2.23 ± 0.57 |
| Lungs | 1.34 ± 0.60 | 2.14 ± 0.65 | 1.63 ± 0.53 |
| Liver | 7.51 ± 2.21 | 14.6 ± 0.54 | 12.8 ± 4.5 |
| Gall Bladder | 5.47 ± 1.85 | 5.18 ± 2.75 | 2.86 ± 1.83 |
| Spleen | 5.02 ± 1.20 | 8.44 ± 0.27 | 5.65 ± 1.73 |
| Pancreas | 2.23 ± 1.96 | 1.39 ± 0.38 | 1.03 ± 0.32 |
| Stomach | 1.68 ± 0.35 | 1.97 ± 0.07 | 1.54 ± 0.17 |
| Small Intestine | 2.22 ± 0.53 | 2.67 ± 0.37 | 1.72 ± 0.38 |
| Large Intestine | 1.90 ± 0.39 | 4.71 ± 0.10 | 3.87 ± 0.67 |
| Kidney | 16.4 ± 2.1 | 21.2 ± 1.87 | 18.1 ± 4.3 |
| Muscle | 1.27 ± 0.45 | 1.23 ± 0.36 | 0.75 ± 0.27 |
| Bone | 3.60 ± 0.71 | 3.32 ± 0.04 | 2.70 ± 0.62 |
| Skin | 1.90 ± 0.91 | 2.18 ± 0.09 | 3.91 ± 2.11 |

Table 3 below illustrates tissue radioactivity distribution of ⁸⁹Zr-PL-HDL in female C57BL/6 (B6) mice bearing orthotopic 4T1 breast cancer tumors. Data presented as mean %ID/g ± SD (n ≥ 3 for each time point).

**Table 3**

| Tissue | 2 h | 24 h | 24 h |
|---|---|---|---|
| Blood | 23.7 ± 1.51 | 2.19 ± 0.23 | 0.49 ± 0.06 |
| Tumor | 3.78 ± 0.50 | 8.55 ± 1.30 | 12.0 ± 4.7 |
| Heart | 1.68 ± 0.10 | 1.26 ± 0.16 | 1.61 ± 0.25 |
| Lungs | 1.58 ± 0.72 | 1.34 ± 0.46 | 1.43 ± 0.50 |
| Liver | 14.1 ± 1.50 | 13.6 ± 2.5 | 15.2 ± 2.5 |
| Gall Bladder | 43.5 ± 19.9 | 2.27 ± 0.91 | 0.69 ± 0.20 |
| Spleen | 7.24 ± 0.44 | 5.37 ± 1.23 | 6.48 ± 1.97 |
| Pancreas | 1.25 ±0.67 | 0.90 ± 0.27 | 0.80 ± 0.24 |
| Stomach | 1.47 ± 0.31 | 1.32 ± 0.02 | 1.14 ± 0.18 |
| Small Intestine | 2.93 ± 0.40 | 1.52 ± 0.06 | 1.77 ± 0.68 |
| Large Intestine | 1.16 ± 0.17 | 1.45 ± 0.04 | 1.28 ± 0.21 |
| Kidney | 13.1 ± 1.7 | 13.2 ± 3.6 | 13.3 ± 2.3 |
| Muscle | 0.99 ± 0.18 | 1.03 ± 0.42 | 0.73 ± 0.30 |
| Bone | 5.80 ± 1.43 | 15.5 ± 1.9 | 17.1 ± 4.8 |
| Skin | 1.90 ± 0.45 | 1.76 ± 0.22 | 2.04 ± 0.99 |

### Example 4: In vivo imaging of radiolabeled HDL nanoparticles

This example demonstrates an exemplary *in vivo* visualization of radiolabeled HDL nanoparticles using PET/CT.

### PET/CT imaging:

Female C57BL/6 (B6) mice (8-10 weeks old, n = 4) bearing 4T1 breast tumors were injected with 6.9 ± 0.1 MBq (range 6.7-7.1 MBq) ⁸⁹Zr-HDL (180-200 µg ApoA1) in 200 µL PBS solution via the lateral tail vein. At 24h the animals were anesthetized with isofluorane (Baxter Healthcare, Deerfield, IL)/oxygen gas mixture (2% for induction, 1 % for maintenance) and a scan was then performed using an Inveon PET/CT scanner (Siemens Healthcare Global, Erlangen, Germany). Whole body PET static scans recording a minimum of 50 million coincident events were performed, with a duration of 15 min. The energy and coincidence timing windows were 350-700 keV and 6 ns, respectively. The image data were normalized to correct for non-uniformity of response of the PET, dead-time count losses, positron branching ratio, and physical decay to the time of injection, but no attenuation, scatter, or partial-volume averaging correction was applied. The counting rates in the reconstructed images were converted to activity concentrations (percentage injected dose [%ID] per gram of tissue) by use of a system calibration factor derived from the imaging of a mouse-sized water-equivalent phantom containing ⁸⁹Zr. Images were analyzed using ASIPro VMTM software (Concorde Microsystems, Knoxville, TN). Quantification of activity concentration was done by averaging the maximum values in at least 5 ROIs drawn on adjacent slices of the tissue of interest. Whole body standard low magnification CT scans were performed with the X-ray tube setup at a voltage of 80 kV and current of 500 µA. The CT scan was acquired using 120 rotational steps for a total of 220 degrees yielding and estimated scan time of 120 s with an exposure of 145 ms per frame.

PET imaging corroborated the observations obtained in *ex vivo* experiments (FIGS. 4A and 4B). The images collected at 24 h show strong liver, kidney, and tumor uptake for both HDL nanoparticles. Quantitative PET data was also in agreement with the biodistribution results discussed in Example 3. Tumor uptake values were measured to be 18.4 ± 2.4 %ID/g (n=2) and 10.6 ± 0.6 %ID/g (n=2) for ⁸⁹Zr-AI-HDL and ⁸⁹Zr-PL-HDL, respectively. Liver and kidney uptake was higher for ⁸⁹Zr-AI-HDL (21.8 ± 4.4 and 19.6 ± 3.8 %ID/g [n=2], respectively) than for ⁸⁹Zr-PL-HDL (18.3 ± 3.1 and 11.6 ± 2.7 %ID/g [n=2]). PET-quantified blood uptake values, measured in the cardiac chambers, were significantly higher than those obtained from *ex vivo* experiments. At this time point, radioactivity in blood was 9.1 ± 1.1 %ID/g for ⁸⁹Zr-AI-HDL and 6.0 ± 0.1 %ID/g for ⁸⁹Zr-PL-HDL. This resulted in increased background signal for ⁸⁹Zr-AI-HDL (FIG. 4B), as well as isolated spots in the intestinal region.

For ⁸⁹Zr-PL-HDL, radioisotope uptake was also observed in the skeleton and joints mirroring *ex vivo* results. Notably, *ex vivo* analysis of tumor sections allowed for the evaluation of the nanoparticles' spatial and cell type distributions.

### Example 5: Ex vivo histological analysis radiolabeled HDL nanoparticles

This example demonstrates an exemplary *ex vivo* analysis of tumor sections to evaluate radiolabeled HDL nanoparticles' spatial and cell type distributions. *Staining*/*Microscopy:*

Tissue sections (10 µm thickness, frozen) were stained for CD31 with anti-CD31 antibodies (Dianova, Hamburg, Germany) followed by a biotinylated goat anti-rat secondary antibody (Vector Labs, Burlingame, CA), streptavidin-HRP D (Ventana Medical Systems, Tucson, AZ) and finally tyramide Alexa Fluor 488 (Invitrogen, Carlsbad, CA). The same sections were stained for Iba1 with anti-Ibal rabbit polyclonal antibody (Wako, Richmond, VA) followed by a biotinylated goat anti-rabbit secondary antibody (VECTASTAIN® ABC kit, Vector Labs, Burlingame, CA), streptavidin-HRP D, and tyramide Alexa Fluor 568 (Invitrogen, Carlsbad, CA) for fluorescent signal (VECTASTAIN® ABC kit, Vector Labs, Burlingame, CA). Additional nuclear staining was performed using 4',6-Diamidino-2-phenylindole dihydrochloride (DAPI, Sigma Aldrich, St. Louis, MO). All sections were counterstained with hematoxylin & eosin (H&E) solution. All images were obtained using an EVOS FL Auto digital inverted fluorescence microscope (Life Technologies, Norwalk, CT). Fluorescent images were obtained at 4× magnification, while brightfield images were obtained at both the 4× and 20× magnification. On stained sections, Iba1 fluorescence was observed using a Texas Red light cube (Ex 585/29, Em 624/40, EVOS LED Light cube), while CD31 fluorescence was observed using a GFP light cube (Ex 470/22, Em 510/42, EVOS LED Light cube).

### Autoradiography:

Following sacrifice, liver, spleen, tumor and muscle tissues were excised and embedded in OCT mounting medium (Sakura Finetek, Torrance, CA), frozen on dry ice, and a series of 10 µm thick frozen sections cut. To determine radiotracer distribution, digital autoradiography was performed by placing tissue sections in a film cassette against a phosphor imaging plate (BASMS-2325, Fujifilm, Valhalla, NY) for 48 h at -20 °C. Phosphor imaging plates were read at a pixel resolution of 25 µm with a Typhoon 7000IP plate reader (GE Healthcare, Pittsburgh, PA). After autoradiographic exposure, the same frozen sections were then used for immunohistochemical staining and imaging.

Histological analysis of tumor sections collected at 24 h allowed us to establish regional distribution of both HDL nanoparticles (FIGS. 5A and 5B). Areas with high ⁸⁹Zr deposition are highly vascularized, as shown by co-localization of CD31 and autoradiography. However, staining for Iba-1 showed that particularly ⁸⁹Zr-PL-HDL also had a high degree of co-localization to macrophage-rich areas (FIG. 5B). Both ⁸⁹Zr-PL-HDL and ⁸⁹Zr-AI-HDL may be accumulated in macrophage-rich regions, as evidenced by the co-localization of radioactivity to Iba-1 positive areas (FIGS. 5A and 5B).

### Example 6: Cellular distribution of radiolabeled HDL nanoparticles

This example demonstrates exemplary flow cytometry analysis of a comprehensive panel of biomarkers to differentiate radiolabeled HDL nanoparticles' cellular specificity for seven different cell types.

### Flow cytometry:

Female C57BL/6 (B6) mice were subcutaneously inoculated with (1×10⁶ cells) murine breast cancer cells (4T1) in their flanks. On day 9 after the implantation, tumors were excised, diced, and digested with a cocktail of enzymes, including liberase TH (Roche), hyaluronidase (Sigma-Aldrich), and DNase (Sigma-Aldrich), in a 37 °C oven for one hour. Single-cell suspension was made by removing tissue aggregates, extracellular matrix, and cell debris from the solution. The same flow cytometry setting was applied to all samples. DiO was detected on FITC channel. All samples were measured on an LSRFortessa (BD Biosciences, San Jose, CA) flow cytometer. Results were analyzed with FlowJo (Ashland, OR) and statistics were calculated with Prism (GraphPad, La Jolla, CA). The antibodies and clones used are listed in Table 4 below and the gating procedure is shown in FIG. 7.

**Table 4**

| Antibody | Clone | Source |
|---|---|---|
| Ly6C | HK1.4 | eBioscience |
| MHCII (I-A/I-E) | M5/114.152 | eBioscience |
| CD45 | 30-F11 | Biolegend |
| CD64 | X54-5/7.1 | Biolegend |
| CD11b | M1/70 | eBioscience |
| CD3 | 17A2 | Biolegend |
| CD31 | 13.3 | BD Biosciences |
| CD11c | M418 | eBioscience |

The intercellular distribution of the ⁸⁹Zr-labeled HDL probes was determined by flow cytometry with a non-radioactive analog labeled with a fluorescent tag (DiO). Using size exclusion chromatography, the labeled nanoparticle DiO@HDL had the same retention time as their unlabeled, plain counterpart and as those of ⁸⁹Zr-AI-HDL and ⁸⁹Zr-PL-HDL.

A flow cytometry gating procedure was used to identify HDL levels in 7 relevant cell types, including TAMs, monocyte-derived cells, monocytes, dendritic cells (DC), T cells, endothelial cells (EC), and tumor cells (FIGS. 6A - 6C). The variations in fluorescence intensities among the different cell types may reflect how HDL nanoparticles interact with their targets. The highest HDL uptake was by TAMs, being 3.5-fold higher than monocyte-derived cells (P < 0.05), 28.8-fold higher than monocytes (P < 0.05), 4.1-folder higher than dendritic cells (P < 0.05), 126-fold higher than T cells (P < 0.05), 8.2-fold higher than endothelial cells (P < 0.05), and 7.2-fold higher than tumor cells (P < 0.05). In this tumor model, TAMs accounted for 13.6 ± 1.7% of live cells and TAM-bound HDL made up 58.6 ± 17.5% of total intracellular HDL in all live cells. The high HDL level in TAMs was the main contributor to a 1.6-fold higher HDL uptake in CD45⁺ immune cells than CD45⁻ non-immune cells (P < 0.05). These data compellingly show that HDL, not only efficiently accumulates in tumors, but also specifically targets TAMs.

Similar to histological analysis in Example 5, HDL preferentially targeted immune cells, particularly macrophages, followed by monocyte-derived cells and dendritic cells. Monocytes, T cells, endothelial cells, and tumor cells were only marginally targeted (FIG. 6C). These results, in conjunction with those observed on histological analysis, indicate that HDL nanoparticles target macrophages with high specificity, in alignment with its biological function.

## Claims

1. A composition comprising a discoidal high density lipoprotein nanoparticle, the nanoparticle comprising Apolipoprotein 1 (ApoAl) and one or more phospholipids, wherein the nanoparticle is radiolabeled with a radioisotope via incorporation of a chelator-modified ApoAl with the radioisotope; and optionally incorporation of a chelator-modified phospholipid with the radioisotope.

2. The composition of claim 1, wherein the nanoparticle is radiolabeled with the radioisotope via the chelator-modified ApoAl, and wherein the nanoparticle comprises the chelator-modified phospholipid.

3. The composition of claim 1 or 2, wherein the nanoparticle has a molecular weight within a range from 100 kDa to 400 kDa as measured by size exclusion chromatography.

4. The composition of any one of the preceding claims, wherein the discoidal high density lipoprotein nanoparticle has average diameter within a range from 5 nm to 30 nm as measured by DLS in water or phosphate-buffered saline (PBS).

5. The composition of any one of the preceding claims, wherein the one or more phospholipids comprise one or more members selected from the group consisting of dimyristoyl phosphatidyl choline (DMPC), 1,2-dipentadecanoyl-sn-glycero-3-phosphocholine, 1,2-dipalmitoyl-sn-glycero-3-phosphocholine (DPPC), 1,2-diheptadecanoyl-sn-glycero-3-phosphocholine, 1,2-distearoyl-sn-glycero-3-phosphocholine (DSPC), 1-myristoyl-2-hydroxy-sn-glycero-3-phosphocholine (MHPC), 1-pentadecanoyl-2-hydroxy-sn-glycero-3-phosphocholine, 1-palmitoyl-2-hydroxy-sn-glycero-3-phosphocholine (PHPC), 1-heptadecanoyl-2-hydroxy-sn-glycero-3-phosphocholine, 1-stearoyl-2-hydroxy-sn-glycero-3-phosphocholine (SHPC), 1-oleoyl-2-hydroxy-sn-glycero-3-phosphocholine (OHPC), 1,2-dioleoyl-sn-glycero-3-phosphocholine (DOPC), and 1,2-dipalmitoleoyl-sn-glycero-3-phosphocholine.

6. The composition of any one of the preceding claims, wherein the chelator-modified phospholipid comprises 1,2-distearoyl-sn-glycero-3-phosphoethanolamine (DSPE).

7. The composition of any one of the preceding claims, wherein some of the one or more phospholipids in the nanoparticle are effectively replaced by the chelator-modified phospholipid.

8. The composition of any one of the preceding claims, wherein the composition comprises a carrier.

9. The composition of any one of claims 1-8, wherein the chelator is a member selected from the group consisting of deferoxamine B (DFO), 1,4,8,1 1-tetraazabicyclo[6.6.2]hexadecane-4,11-diyl)diacetic acid (CB-TE2A); diethylenetriaminepentaacetic acid (DTPA); 1,4,7, 10-tetraazacyclotetradecane- 1,4,7, 10-tetraacetic acid (DOTA); thylenediaminetetraacetic acid (EDTA); ethylene glycolbis(2-aminoethyl)-N,N,N',N'- tetraacetic acid (EGTA); 1,4,8,1 1-tetraazacyclotetradecane-1,4,8,1 1-tetraacetic acid (TETA); ethylenebis-(2-4 hydroxy-phenylglycine) (EHPG); 5-Cl-EHPG; 5Br-EHPG; 5- Me-EHPG; 5t-Bu-EHPG; 5-sec-Bu-EHPG; benzodiethylenetriamine pentaacetic acid (benzo-DTPA); dibenzo-DTPA; phenyl-DTPA, diphenyl-DTPA; benzyl-DTPA; dibenzyl DTPA; bis-2 (hydroxybenzyl)-ethylene-diaminediacetic acid (HBED) and derivatives thereof; Ac-DOTA; benzo-DOTA; dibenzo-DOTA; 1,4,7-triazacyclononane N,N',N"- triacetic acid (NOTA); benzo-NOTA; benzo-TETA, Octadentate Hydroxypyridinonate (HOPO) ligands, benzo-DOTMA, where DOTMA is 1,4,7, 10-tetraazacyclotetradecane-1,4,7,10-tetra(methyl tetraacetic acid), benzo-TETMA wherein TETMA is 1,4,8,1 1-tetraazacyclotetradecane-1,4,8,1 1-(methyl tetraacetic acid)); derivatives of 1,3-propylenediaminetetraacetic acid (PDTA); triethylenetetraaminehexaacetic acid (TTHA); derivatives of 1,5,10-N,N',N"-tris(2,3- dihydroxybenzoyl)-tricatecholate (LICAM); and 1,3,5-N,N',N"-tris(2,3- dihydroxybenzoyl)aminomethylbenzene (MECAM).

10. The composition of any one of claims 1-9, wherein the chelator comprises deferoxamine B (DFO).

11. The composition of any one of claims 1-10, wherein the radioisotope comprises a member selected from the group consisting of ⁸⁹Zr, ^{99m}Tc, ¹¹¹In, ⁶⁴Cu, ⁶⁷Ga, ¹⁸⁶Re, ¹⁸⁸Re, ¹⁵³Sm, ¹⁷⁷Lu, ⁶⁷Cu, ¹²³I, ¹²⁴I, ¹²⁵I, ¹¹C, ¹³N, ¹⁵O, ¹⁸F, ¹⁸⁶Re, ¹⁸⁸Re, ¹⁵³Sm, ¹⁶⁶Ho, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, and ¹⁹²Ir.

12. The composition of any one of claims 1-11, wherein the radioisotope comprises ⁸⁹Zr.

13. A composition of any one of claims 1-12, for use in a method of imaging tumor-associated macrophages (TAMs) by high density lipoprotein positron emission tomography (HDL PET), the method comprising:
administering to a subject the composition to allow the nanoparticle to accumulate in a region of TAMs; and
detecting the radioisotope via PET after accumulation of the nanoparticle in the region of TAMs.

14. A method of making the discoidal high density lipoprotein nanoparticle of claim 1, the method comprising one or more of (i), (ii), (iii) and (iv):
(i) modifying ApoAl with a chelator, reacting the modified ApoAl with a radioisotope comprising an oxalate moiety, and incorporating the radiolabeled chelator-modified ApoAl in the nanoparticle;
(ii) modifying ApoAl with a chelator, incorporating the chelator-modified ApoAl in the nanoparticle, and reacting the chelator-modified ApoAl with a radioisotope comprising an oxalate moiety;
(iii) modifying DSPE with a chelator, reacting the modified DSPE with a radioisotope comprising an oxalate moiety , and incorporating the radiolabeled chelator-modified DSPE in the nanoparticle; and
(iv) modifying DSPE with a chelator, incorporating the chelator-modified DSPE in the nanoparticle, and reacting the chelator-modified DSPE with a radioisotope comprising an oxalate moiety.

15. A composition of any one of claims 1-12 for use in a method of assessing prognosis and therapy outcome of different stages of a disease in a subject.

## Patentansprüche

1. Zusammensetzung, umfassend ein scheibenförmiges Nanoteilchen aus Lipoprotein hoher Dichte, wobei das Nanoteilchen Apolipoprotein 1 (ApoA1) und ein oder mehrere Phospholipide umfasst, wobei das Nanoteilchen mit einem Radioisotop durch Einbau eines Chelator-modifizierten ApoA1 mit dem Radioisotop und optionalen Einbau eines Chelator-modifizierten Phospholipid mit dem Radioisotop radioaktiv markiert ist.

2. Zusammensetzung nach Anspruch 1, wobei das Nanoteilchen mit dem Radioisotop durch das Chelator-modifizierte ApoA1 radioaktiv markiert ist, und wobei das Nanoteilchen das Chelator-modifizierte Phospholipid umfasst.

3. Zusammensetzung nach Anspruch 1 oder 2, wobei das Nanoteilchen ein Molekulargewicht von 100 kDa bis 400 kDa aufweist, wie durch Größenausschlusschromatographie gemessen.

4. Zusammensetzung nach einem der vorhergehenden Ansprüche, wobei das scheibenförmige Nanoteilchen aus Lipoprotein hoher Dichte einen mittleren Durchmesser innerhalb eines Bereichs von 5 nm bis 30 nm aufweist, wie durch DLS in Wasser oder phosphatgepufferter Kochsalzlösung (PBS) gemessen.

5. Zusammensetzung nach einem der vorhergehenden Ansprüche, wobei das eine oder die mehreren Phospholipide ein oder mehrere Elemente umfassen, die ausgewählt sind aus der Gruppe bestehend aus Dimyristoylphosphatidylcholin (DMPC), 1,2-Dipentadecanoyl-sn-glycero-3-phosphocholin, 1,2-Dipalmitoyl-sn-glycero-3-phosphocholin (DPPC), 1,2-Diheptadecanoyl-sn-glycero-3-phosphocholin, 1,2-Distearoyl-sn-glycero-3-phosphocholin (DSPC), 1-Myristoyl-2-hydroxy-sn-glycero-3-phosphocholin (MHPC), 1-Pentadecanoyl-2-hydroxy-sn-glycero-3-phosphocholin, 1-Palmitoyl-2-hydroxy-sn-glycero-phosphocholin (PHPC), 1-Heptadecanoyl-2-hydroxy-sn-glycero-3-phosphocholin, 1-Stearoyl-2-hydroxy-n-glycero-3-phosphocholin (SHPC), 1-Oleoyl-2-hydroxy-sn-glycero-3-phosphocholin (OHPC), 1,2-Dioleoyl-sn-glycero-3-phosphocholin (DOPC) und 1,2-Dipalmitoleoyl-sn-glycero-3-phosphocholin.

6. Zusammensetzung nach einem der vorhergehenden Ansprüche, wobei das Chelator-modifizierte Phospholipid 1,2-Distearoyl-sn-glycero-3-phosphoethanolamin (DSPE) umfasst.

7. Zusammensetzung nach einem der vorhergehenden Ansprüche, wobei einige des einen oder der mehreren Phospholipide im Nanoteilchen durch das Chelator-modifizierte Phospholipid wirksam ersetzt werden.

8. Zusammensetzung nach einem der vorhergehenden Ansprüche, wobei die Zusammensetzung einen Träger umfasst.

9. Zusammensetzung nach einem der Ansprüche 1 bis 8, wobei der Chelator ein Element ist, das ausgewählt ist aus der Gruppe bestehend aus Deferoxamin B (DFO), 1,4,8,11-Tetraazabicyclo[6.6.2]hexadecan-4,11-diyl)diessigsäure (CB-TE2A); Diethylentriaminpentaessigsäure (DTPA); 1,4,7,10-Tetraazacyclotetradecan-1,4,7,10-tetraessigsäure (DOTA); Ethylendiamintetraessigsäure (EDTA); Ethylenglycol-bis(2-aminoethyl)-N,N,N',N'-tetraessigsäure (EGTA); 1,4,8,11-Tetraazacyclotetradecan-1,4,8,11-tetraessigsäure (TETA); Ethylen-bis-(2-4-hydroxphenylglycin) (EHPG); 5-C1-EHPG; 5Br-EHPG; 5-Me-EHPG; 5t-Bu-EHPG; 5-sec-Bu-EHPG; Benzodiethylentriaminpentaessigsäure (Benzo-DTPA); Dibenzo-DTPA; Phenyl-DTPA, Diphenyl-DTPA; Benzyl-DTPA; Dibenzyl-DTPA; Bis-2-(hydroxybenzyl)-ethylen-diamindiessigsäure (HBED) und Derivativen davon; Ac-DOTA; Benzo-DOTA; Dibenzo-DOTA; 1,4,7-Triazacyclononan-N,N',N"-triessigsäure (NOTA); Benzo-NOTA; Benzo-TETA, Octadentat-Hydroxypyridinonat (HOPO)-Liganden, Benzo-DOTMA, wobei DOTMA 1,4,7,10-Tetraazacyclotetradecan-1,4,7,10-tetra(methyltetraessigsäure) ist, Benzo-TETMA, wobei TETMA 1,4,8,11-Tetraazacyclotetradecan-1,4,8,11-(methyltetraessigsäure) ist; Derivaten von 1,3-Propylendiamintetraessigsäure (PDTA); Triethylentetraaminhexaessigsäure (TTHA); Derivaten von 1,5,10-N,N',N"-tris(2,3-Dihydroxybenzoyl)-tricatecholat (LICAM); und 1,3,5-N,N',N"-tris(2,3-Dihydroxybenzoyl)aminomethylbenzol (MECAM).

10. Zusammensetzung nach einem der Ansprüche 1 bis 9, wobei der Chelator Deferoxamin B (DFO) umfasst.

11. Zusammensetzung nach einem der Ansprüche 1 bis 10, wobei das Radioisotop ein Element umfasst, das ausgewählt ist aus der Gruppe bestehend aus ⁸⁹Zr, ^{99m}Tc, ¹¹¹In, ⁶⁴Cu, ⁶⁷Ga, ¹⁸⁶Re, ¹⁸⁸Re, ¹⁵³Sm, ¹⁷⁷Lu, ⁶⁷Cu, ¹²³I, ¹²⁴I, ¹²⁵I, ¹¹C, ¹³N, ¹⁵O, ¹⁸F, ¹⁸⁶Re, ¹⁸⁸Re, ¹⁵³Sm, ¹⁶⁶HO, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag und ¹⁹²Ir.

12. Zusammensetzung nach einem der Ansprüche 1 bis 11, wobei das Radioisotop ⁸⁹Zr umfasst.

13. Zusammensetzung nach einem der Ansprüche 1 bis 12 zur Verwendung in einem Verfahren zur Abbildung von tumorassoziierten Makrophagen (TAMs) durch Positron-Emissions-Tomographie (HDL-PET) unter Verwendung von Lipoproteinen hoher Dichte, wobei das Verfahren umfasst:
Verabreichen der Zusammensetzung an einen Patienten, um zu ermöglichen, dass das Nanoteilchen sich in einer Region von TAMs ansammelt; und
Nachweisen des Radioisotops durch PET nach der Anhäufung des Nanoteilchens in der Region von TAMs.

14. Verfahren zur Herstellung des scheibenförmigen Nanoteilchens aus Lipoprotein hoher Dichte nach Anspruch 1, wobei das Verfahren eines oder mehrere von (i), (ii), (iii) und (iv) umfasst:
(i) Modifizieren von ApoA1 mit einem Chelator, Umsetzen des modifizierten ApoA1 mit einem Radioisotop, das einen Oxalatrest umfasst, und Einbauen des radioaktiv markierten Chelator-modifizierten ApoA1 in das Nanoteilchen;
(ii) Modifizieren von ApoA1 mit einem Chelator, Einbauen des Chelator-modifizierten ApoA1 in das Nanoteilchen und Umsetzen des Chelator-modifizierten ApoA1 mit einem Radioisotop, das einen Oxalatrest umfasst;
(iii) Modifizieren von DSPE mit einem Chelator, Umsetzen des modifizierten DSPE mit einem Radioisotop, das einen Oxalatrest umfasst, und Einbauen des radioaktiv markierten Chelator-modifizierten DSPE in das Nanoteilchen; und
(iv) Modifizieren von DSPE mit einem Chelator, Einbauen des Chelator-modifizierten DSPE in das Nanoteilchen und Umsetzen des Chelator-modifizierten DSPE mit einem Radioisotop, das einen Oxalatrest umfasst.

15. Zusammensetzung nach einem der Ansprüche 1 bis 12 zur Verwendung in einem Verfahren zur Auswertung eines Prognose- und Therapieergebnisses von verschiedenen Phasen einer Krankheit eines Patienten.

## Revendications

1. Composition comprenant une nanoparticule de lipoprotéine haute densité discoïdale, la nanoparticule comprenant de l'apolipoprotéine 1 (ApoA1) et un ou plusieurs phospholipides, dans laquelle la nanoparticule est radiomarquée par un isotope radioactif via l'incorporation d'une ApoA1 modifiée par un chélateur avec l'isotope radioactif ; et éventuellement l'incorporation d'un phospholipide modifié par un chélateur avec l'isotope radioactif.

2. Composition selon la revendication 1, dans laquelle la nanoparticule est radiomarquée par l'isotope radioactif via l' ApoA1 modifiée par un chélateur, et dans laquelle la nanoparticule comprend le phospholipide modifié par un chélateur.

3. Composition selon la revendication 1 ou 2, dans laquelle la nanoparticule a un poids moléculaire situé dans la plage allant de 100 kDa à 400 kDa, tel que mesuré par chromatographie d'exclusion par la taille.

4. Composition selon l'une quelconque des revendications précédentes, dans laquelle la nanoparticule de lipoprotéine haute densité discoïdale a un diamètre moyen situé dans la plage allant de 5 nm à 30 nm, telle que mesurée par DLS dans de l'eau ou de la solution saline tamponnée au phosphate (PBS).

5. Composition selon l'une quelconque des revendications précédentes, dans laquelle les uns ou plusieurs phospholipides comprennent un ou plusieurs membres choisis dans le groupe constitué par la dimyristoyl-phosphatidylcholine (DMPC), la 1,2-dipentadécanoyl-sn-glycéro-3-phosphocholine, la 1,2-dipalmitoyl-sn-glycéro-3-phosphocholine (DPPC), la 1,2-diheptadécanoyl-sn-glycéro-3-phosphocholine, la 1,2-distéaroyl-sn-glycéro-3-phosphocholine (DSPC), la 1-myristoyl-2-hydroxy-sn-glycéro-3-phosphocholine (MHPC), la 1-pentadécanoyl-2-hydroxy-sn-glycéro-3-phosphocholine, la 1-palmitoyl-2-hydroxy-sn-glycéro-3-phosphocholine (PHPC), la 1-heptadécanoyl-2-hydroxy-sn-glycéro-3-phosphocholine, la 1-stéaroyl-2-hydroxy-sn-glycéro-3-phosphocholine (SHPC), la 1-oléoyl-2-hydroxy-sn-glycéro-3-phosphocholine (OHPC), la 1,2-dioléoyl-sn-glycéro-3-phosphocholine (DOPC), et la 1,2-dipalmitoléoyl-sn-glycéro-3-phosphocholine.

6. Composition selon l'une quelconque des revendications précédentes, dans laquelle le phospholipide modifié par un chélateur comprend de la 1,2-distéaroyl-sn-glycéro-3-phosphoéthanolamine (DSPE).

7. Composition selon l'une quelconque des revendications précédentes, dans laquelle certains parmi les uns ou plusieurs phospholipides dans la nanoparticule sont efficacement remplacés par le phospholipide modifié par un chélateur.

8. Composition selon l'une quelconque des revendications précédentes, dans laquelle la composition comprend un véhicule.

9. Composition selon l'une quelconque des revendications 1 à 8, dans laquelle le chélateur est un membre choisi dans le groupe constitué par la déféroxamine B (DFO), l'acide 1,4,8,11-tétraazabicyclo[6.6.2]hexadécane-4,11-diyl)diacétique (CB-TE2A) ; l'acide diéthylènetriaminepentaacétique (DTPA) ; l'acide 1,4,7,10-tétraazacyclotétradécane-1,4,7,10-tétraacétique (DOTA) ; l'acide éthylènediaminetétraacétique (EDTA), l'acide éthylèneglycolbis(2-aminoéthyl)-N,N,N',N'-tétraacétique (EGTA) ; l'acide 1,4,8,11-tétraazacyclotétradécane-1,4,8,1 1-tétraacétique (TETA) ; l'éthylènebis(2,4-hydroxyphénylglycine) (EHPG) ; la 5-C1-EHPG ; la 5-Br-EHPG ; la 5-Me-EHPG ; la 5-t-Bu-EHPG ; la 5-sec-Bu-EHPG ; l'acide benzodiéthylènetriaminepentaacétique (benzo-DTPA) ; le dibenzo-DTPA ; le phényl-DTPA, le diphényl-DTPA ; le bentyl-DTPA ; le dibenzyl-DTPA ; l'acide bis-2(hydroxybenzyl)éthylènediaminediacétique (HBED) et ses dérivés ; l'Ac-DOTA ; le benzo-DOTA ; le dibenzo-DOTA ; l'acide 1,4,7-triazacyclononane-N,N',N"-triacétique (NOTA) ; le benzo-NOTA ; le benzo-TETA, les ligands hydroxypyridinonate octadenté (HOPO), le benzo-DOTMA, où le DOTMA est l'acide 1,4,7,10-tétraazacyclotétradécane-1,4,7,10-tétra(méthyltétraacétique), le benzo-TETMA où le TETMA est l'acide 1,4,8,11-tétraazacyclotétradécane-1,4,8,1 1-(méthyltétraacétique) ; les dérivés d'acide 1,3-propylènediaminetétraacétique (PDTA) ; l'acide triéthylènetétraminehexaacétique (TTHA) ; les dérivés de tricatécholate de 1,5,10-N,N',N"-tris(2,3-dihydroxybenzoyle) (LICAM) ; et le 1,3,5-N,N',N"-tris(2,3-dihydroxybenzoyl)aminométhylbenzène (MECAM).

10. Composition selon l'une quelconque des revendications 1 à 9, dans laquelle le chélateur comprend de la déféroxamine B (DFO).

11. Composition selon l'une quelconque des revendications 1 à 10, dans laquelle l'isotope radioactif comprend un membre choisi dans le groupe constitué par ⁸⁹Zr, ^{99m}Tc, ¹¹¹In, ⁶⁴Cu, ⁶⁷Ga, ¹⁸⁶Re, ¹⁸⁸Re, ¹⁵³Sm, ¹⁷⁷Lu, ⁶⁷Cu, ¹²³I, ¹²⁴I, ¹²⁵I, ¹¹C, ¹³N, ¹⁵O, ¹⁸F, ¹⁸⁶Re, ¹⁸⁸Re, ¹⁵³Sm, ¹⁶⁶HO, ¹⁷⁷Lu, ¹⁴⁹Pm, ⁹⁰Y, ²¹²Bi, ¹⁰³Pd, ¹⁰⁹Pd, ¹⁵⁹Gd, ¹⁴⁰La, ¹⁹⁸Au, ¹⁹⁹Au, ¹⁶⁹Yb, ¹⁷⁵Yb, ¹⁶⁵Dy, ¹⁶⁶Dy, ⁶⁷Cu, ¹⁰⁵Rh, ¹¹¹Ag, et ¹⁹²Ir.

12. Composition selon l'une quelconque des revendications 1 à 11, dans laquelle l'isotope radioactif comprend ⁸⁹Zr.

13. Composition selon l'une quelconque des revendications 1 à 12, destinée à être utilisée dans une méthode d'imagerie de macrophages associés à une tumeur (TAM) par tomographie par émission de positons de lipoprotéine haute densité (HDL PET), la méthode comprenant :
l'administration à un sujet de la composition pour que la nanoparticule puisse s'accumuler dans une région de TAM ; et
la détection de l'isotope radioactif via PET après accumulation de la nanoparticule dans la région de TAM.

14. Procédé de préparation de la nanoparticule de lipoprotéine haute densité discoïdale selon la revendication 1, le procédé comprenant un ou plusieurs parmi (i), (ii), (iii) et (iv) :
(i) modification d'ApoA1 avec un chélateur, réaction de l'ApoA1 modifiée avec un isotope radioactif comprenant un élément oxalate, et incorporation de l'ApoA1 modifiée par un chélateur et radiomarquée dans la nanoparticule ;
(ii) modification d'ApoA1 avec un chélateur, incorporation de l'ApoA1 modifiée par un chélateur dans la nanoparticule et réaction de l'ApoA1 modifiée par un chélateur avec un isotope radioactif comprenant un élément oxalate ;
(iii) modification de la DSPE avec un chélateur, réaction de la DSPE modifiée avec un isotope radioactif comprenant un élément oxalate, et incorporation de la DSPE modifiée par un chélateur et radiomarquée dans la nanoparticule ; et
(iv) modification de la DSPE avec un chélateur, incorporation de la DSPE modifiée par un chélateur dans la nanoparticule et réaction de la DSPE modifiée par un chélateur avec un isotope radioactif comprenant un élément oxalate.

15. Composition selon l'une quelconque des revendications 1 à 12, destinée à être utilisée dans une méthode de détermination d'un pronostic et d'un résultat thérapeutique de différents stades d'une maladie chez un sujet.
